(19) Europäisches Patentamt
European Patent Office
Office européen des brevets

(11) **EP 4 110 410 B1**

(12) # EUROPEAN PATENT SPECIFICATION

(45) Date of publication and mention
of the grant of the patent:
**18.12.2024 Bulletin 2024/51**

(21) Application number: **21708610.7**

(22) Date of filing: **25.02.2021**

(51) International Patent Classification (IPC):
**A61L 15/42** (2006.01)  **A61L 15/58** (2006.01)
**A61L 24/00** (2006.01)  **A61L 24/04** (2006.01)
**C08G 18/08** (2006.01)  **C08G 18/10** (2006.01)
**C08G 18/22** (2006.01)  **C08G 18/24** (2006.01)
**C08G 18/28** (2006.01)  **C08G 18/48** (2006.01)
**C08G 18/67** (2006.01)  **C08G 18/73** (2006.01)
**C08G 18/79** (2006.01)  **C08L 75/14** (2006.01)
**C09J 175/14** (2006.01)

(52) Cooperative Patent Classification (CPC):
(C-Sets available)
**A61L 15/42; A61L 15/58; A61L 24/001;
A61L 24/046; C08G 18/0852; C08G 18/10;
C08G 18/222; C08G 18/246; C08G 18/2825;
C08G 18/4829; C08G 18/672; C08G 18/73;
C08G 18/792; C08L 75/14; C09J 175/14**      (Cont.)

(86) International application number:
**PCT/EP2021/054666**

(87) International publication number:
**WO 2021/170711 (02.09.2021 Gazette 2021/35)**

(54) **SWITCHABLE ADHESIVE COMPOSITIONS**

WECHSELBARE KLEBSTOFFZUSAMMENSETZUNGEN

COMPOSITIONS ADHÉSIVES COMMUTABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.02.2020 GB 202002659**

(43) Date of publication of application:
**04.01.2023 Bulletin 2023/01**

(73) Proprietor: **Lumina Adhesives AB
41265 Göteborg (SE)**

(72) Inventors:
• **TUNIUS, Mats
443-39 Lerum (SE)**
• **BOSAEUS, Niklas
414-78 Goteborg (SE)**

(74) Representative: **Gill Jennings & Every LLP
The Broadgate Tower
20 Primrose Street
London EC2A 2ES (GB)**

(56) References cited:
**WO-A1-00/61692          WO-A1-2015/132551
US-A1- 2018 030 321**

EP 4 110 410 B1

(52) Cooperative Patent Classification (CPC): (Cont.)

C-Sets
**A61L 15/58, C08L 75/04;**
**A61L 24/046, C08L 75/04;**
**C08G 18/672, C08G 18/48**

2

**Description**

**Introduction**

This invention relates to switchable adhesive compositions, and more particularly to polyurethane-based pressure sensitive adhesive compositions that comprise curable moieties. The invention also relates to methods for producing the adhesive compositions and to articles comprising the switchable adhesive compositions.

The adhesive compositions of the invention are "switchable" from a tacky state to a non-tacky state by initiating curing of the curable moieties. The result of this curing is a reduction in the tackiness of the adhesive, as evidenced by a reduction in the peel strength of the switched adhesive as compared to a non-switched adhesive.

The adhesive compositions of the invention are useful for various applications in which an article needs to be adhered to a surface for a time and subsequently removed. Although the adhesive compositions of the invention may be used with various substrates, they are particularly useful for applications where a strong bond to a delicate surface is required.

In particular, the adhesive compositions of the invention find utility in adhesive medical products that are applied to the skin of a patient, since switching of the adhesive composition to a low tack state allows the medical product to be removed with less pain and/or damage to the underlying tissue. Such medical products are of particular use for patients with injuries or long-term medical condition requiring repeated application of dressings, as well as for patients (e.g. babies, especially neonatal, and the elderly) with sensitive skin.

**Background of the Invention**

Many types of medical products, such as surgical or medical dressings and bandages, comprise a layer of a pressure sensitive adhesive to enable the medical product to be attached to the skin of a patient. Adhesives used for this purpose must be sufficiently strong to form a strong bond to the skin that is able to resist peeling or accidental removal while the medical product is in use. However, conventional adhesives having these properties can cause localised trauma and/or pain to the patient when the dressing or bandage is removed from the skin. This is particularly true for patients with a long term condition that requires an adhesive dressing to be applied to the same part of the body repeatedly over a prolonged period, such as stoma patients. It is also true for patients with fragile skin, especially the elderly and children.

Therefore, a need has been identified to provide "switchable" pressure sensitive adhesives that have high initial peel strength, but that can be switched to a form with significantly lower peel strength when removal of an adhered product is required. Such adhesives would allow medical products to be attached securely to the skin in use, but to be removed easily and without causing localised trauma and/or pain to a patient upon removal. To have practical utility for adhesive medical products it is furthermore necessary that a reduction in peel strength can be achieved in a controlled manner in a relatively short time, say from a number of seconds to at most a few minutes.

For convenience, the term "switchable" will be used to refer to adhesive compositions which can be changed from a tacky to a non-tacky state or, more accurately, to a very low-tack state. Recognizing that the expression "low-tack" is a relative term, it will be defined herein as meaning the condition of minimum tackiness which the adhesive composition reaches after switching from its tacky state. The reduction in peel force may be as great as 99% or as little as 30%. Typically, the reduction in peel force is around 99% on HDPE and around 90% on skin.

A form of switchable adhesive is disclosed in US 5,032,637, US 5,352,516, US 4,331,576 and US 5,182,323. These documents describe adhesives that become less tacky upon contact with water. However, such adhesives are unsuitable if used on a wound dressing and the patient's wound needs to be kept dry.

US 2013/0123678, WO 2010/129299 and WO 2013/066401 disclose multilayer adhesive laminates that are selectively releasable from a substrate upon application of an agent to an outer face or perimeter of the laminate. The laminate comprises an interior layer and a plurality of fluid passageway conduits or apertures extending through the interior layer, an adhesive layer disposed along the bottom surface of the interior layer and a carrier layer disposed on the top surface of the interior layer. Removal of the carrier layer and application of an effective amount of the agent results in transport of the agent to the adhesive. After sufficient contact time between the agent and the adhesive, the adhesively adhered laminate can be removed from the substrate.

EP 0863775, US 6,184,264 and US 6,610,762 disclose adhesives that are switchable when exposed to visible light or low dosages of UV light. The switchable adhesives described in these documents generally comprise an acrylic adhesive based on copolymers of alkyl acrylates, acrylic acid and/or a free radical polymerisable vinyl moiety "modified" or functionalised by a curable moiety bound thereto. Typical of the bound-in curable moieties are those derived from anthracenes, cinnamates, maleimides, coumarins, acrylates and/or methacrylates.

US 4,999,242 discloses a radiation-curable tape comprising an adhesive curable by irradiation, for example by UV or ionising radiation such as an electron beam, disposed on a radiation transmitting-substrate. The radiation-curable adhesive is composed of an acrylic adhesive, a compound having carbon-carbon double bonds and a silicone acrylate compound. The radiation-curable tape can be used in processing steps for the production of semiconductor wafers, ceramics and glass employing a direct picking-up system.

US 5,955,512 discloses a pressure sensitive adhesive composition comprising an acrylic copolymer (A), an energy beam polymerizable urethane acrylate oligomer (B) and an energy beam polymerizable compound having one acryloyl group or methacryloyl group in each molecule thereof (C). The composition preferably also contains a plasticizer (D), a crosslinking agent (E) and/or a photo-polymerization initiator (F) according to necessity.

WO 2016/124339 A1 by the current inventors discloses switchable adhesive compositions based on polyurethane adhesives. Unsaturated curable molecules may be mixed in and/or bound to the polyurethane polymer backbone. Photoinitiated curing of the curable molecules results in the formation of a crosslinked network which results in a reduction in peel strength of the adhesive.

Despite the developments described above, there remains a need in the art for improved curable adhesives, which exhibit the properties of high peel strength before switching, low peel strength after switching, and low switching time once switching is initiated. It is found that adhesive formulations with good adhesive properties often demonstrate poor switching capability, and adhesive formulations with good switching capability often demonstrate low peel strength and/or low cohesiveness (the adhesive has low internal strength such that an adhered material will detach from a surface leaving residues of the adhesive on the surface). Obtaining high peel strength, high cohesiveness and good switching capability simultaneously is therefore a particular challenge when developing switchable adhesives.

Further desirable properties of switchable adhesives include stability in the unswitched state, both in storage and when adhered to a substrate, and ease of manufacture without complex synthetic steps, long processing times, or premature activation of the switching process during handling of the adhesive in assembly lines.

## Summary of the Invention

The invention has been devised in view of the disadvantages of known switchable pressure sensitive adhesive systems as set out above and provides an improved switchable adhesive composition having high peel strength, high cohesiveness, fast switching times, and a low switched peel strength.

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

In a first aspect, the invention provides an adhesive polyurethane composition comprising the reaction product of:

(A) a polymer component having a weight average molecular weight in the range of 500 to 100,000 Dalton, and containing an average per molecule of X nucleophilic functional groups containing an active hydrogen atom, wherein X represents a number having a value of at least 2; and
(B) a cross-linking component obtained by reacting:

(i) a polyisocyanate component having an average of at least Y isocyanate functions per molecule, wherein Y represents a number in the range from 1.8 to 6;
(ii) at least one compound comprising a functional group that is curable by free-radical polymerisation and further comprising a nucleophilic functional group containing an active hydrogen atom; and
(iii) at least one compound comprising a nucleophilic functional group containing an active hydrogen atom and which does not include a functional group that is curable by free-radical polymerisation;

wherein the total degree of substitution of the polyisocyanate component (i) by the compounds (ii) and (iii) is at least 0.2 and no more than the lesser of 0.3Y and 0.8.

The cross-linking component (B) is therefore the product of the reaction between a polyisocyanate component (i) and the compound (ii) that comprises a curable functional group, and the compound (iii) which does not comprise a curable functional group. The total degree of substitution of the polyisocyanate component (i) is controlled within the specified ranges such that at least a portion of the isocyanate groups remain unreacted. The unreacted isocyanate groups are available to react with the nucleophilic functional groups of the polymer component (A) to form a cross-linked adhesive network including pendant curable groups.

The degree of substitution of the polyisocyanate component (i) represents the fraction of isocyanate groups of the polyisocyanate component (i) that are substituted by the nucleophilic functional groups containing an active hydrogen atom from compounds (ii) and (iii). It may be defined as follows:

$$\text{degree of substitution} = \frac{\text{total moles nucleophilic groups in compound (ii) and compound (iii)}}{\text{total moles isocyanate groups in polyisocyanate component (i)}}$$

The total degree of substitution of the polyisocyanate component (i) relates to the total degree of substitution by both compound (ii) and compound (iii).

As a result of the partial reaction of the polyisocyanate component (i) with the compound (ii) and also with the compound (iii), a product mixture is obtained that comprises a statistical mixture of substituted polyisocyanates. Some individual polyisocyanate molecules will be substituted by compound (ii) and/or (iii) at all of the isocyanate groups, whereas other polyisocyanate molecules will not be substituted at all. Still other polyisocyanate molecules will include one or more substituted isocyanate groups and one or more unsubstituted isocyanate groups in the same molecule. The unsubstituted isocyanate groups are therefore available for reaction with the nucleophilic functional groups of the polymer component (A).

In the case that an individual polyisocyanate molecule is fully substituted by compounds (ii) and/or (iii), the resulting compound will have no free isocyanate groups and will not be able to react with the polymer component (A). However, that compound may function as an unbound curable molecule which is able to participate in the curing reaction to switch the adhesive from a high-tack to a low-tack state.

If an individual polyisocyanate molecule (i) is partially substituted by compounds (ii) and/or (iii), or is unsubstituted, it will be able to react with the nucleophilic functional groups of the polymer component and will thus become bound to the polyurethane adhesive. Compounds in the cross-linking component (B) having only one unsubstituted isocyanate group will form a terminal urethane/urea/amide bond to the polymer component and thus function to bind curable molecules to the polyurethane adhesive. Compounds in the cross-linking component (B) having two unsubstituted isocyanate groups are able to cross-link two end groups of the polymer component so as to form a crosslinked polymeric network that is characteristic of polyurethanes.

Compounds in the cross-linking component (B) having three (or more) unsubstituted isocyanate groups are able to crosslink three (or more) end groups of the polymer component and thus form a nodal (or branching) point in the polyurethane network where multiple polymer end groups are bound to the same polyisocyanate molecule.

It has been found that polyurethane-based adhesives formed from a mixture of polyisocyanates having different degrees of substitution results in an improved adhesive. Polyurethane adhesives are typically formed by reaction of a polymer component with only one type of polyisocyanate molecule, typically a diisocyanate. As a result, the polymer chains are simply cross-linked by the diisocyanates and therefore the adhesive contains only this type of cross-linking.

By contrast, in accordance with the invention, the polymer component (A) is reacted with a variety of different compounds containing different numbers of isocyanate groups, resulting in a variety of different bonding modes of the polymer component (A). As a result, an adhesive is obtained with improved adhesiveness and cohesiveness. This simultaneously provides the advantage that a substantial portion of the curable molecules are bound to the polyurethane backbone, which results in an adhesive having very low cytotoxicity.

The adhesive polyurethane compositions of the invention generally have a gel-like consistency with a relatively low density of cross-linking. The compositions are capable of forming polar bonds and Van der Waals bonds with substrates, and this give the adhesive composition its tackiness. When a curing (or switching) reaction is initiated, for example, by exposure to long wavelength UV or visible light in the presence of a photoinitiator, the curable groups from the compound (ii) form chemical bonds with other curable groups. This curing reaction significantly increases the cross-linking density within the adhesive composition, compared to the unswitched adhesive composition. This reduces the mobility and free volume of the polymeric segments of the adhesive composition, meaning that the composition loses its flowability, essentially becoming an elastic film with no or little tackiness.

When a conventional pressure sensitive adhesive is peeled from a surface to which it is adhered, the required energy may be about $10^2$ to $10^4$ times larger than that which would be needed from a thermodynamic perspective, as a result of internal energy losses in the adhesive bulk material and because at least a component of the peeling force acts in the direction that is normal to the surface. In the switchable adhesive composition of the invention, the dense polymeric network that is formed by the curable functional groups upon switching of the adhesive reduces the tackiness and flexibility of the bulk material of the composition. The required peel force is therefore reduced to a value that is closer to that which is implied by thermodynamic considerations alone.

## Detailed Description of the Invention

The adhesive polyurethane compositions are obtained by the reaction between a polymer component (A) (e.g. a polyether or polyester) with a cross-linking component (B).

**Polymer component (A)**

The adhesive polyurethane compositions of the invention are formed from the reaction of a polymer component (A) and a cross-linking component (B), wherein the polymer component has a weight average molecular weight in the range of 500 to 100,000 Dalton, and contains an average per molecule of X nucleophilic functional groups containing an active hydrogen atom, wherein X represents a number having a value of at least 2. The nucleophilic functional groups of the polymer component (A) react with the free isocyanate groups of the cross-linking component (B) to form a cross-linked polyurethane adhesive network.

X preferably has a value of at least 2.2, preferably at least 2.4, preferably at least 2.6, preferably at least 2.8, preferably at least 3. In particular, it is preferred that at least a portion of the polymer molecules in the polymer component (A) contain three or more nucleophilic functional groups. Polymer molecules having 3 or more nucleophilic functional groups are able to introduce further cross-linking into the polymeric network of the polyurethane adhesive which contributes to improved adhesive performance.

The polymer component (A) is preferably selected from hydroxy-terminated polymers/oligomers. For example the polymer component (A) may be selected from hydroxy-terminated polyethers, hydroxy-terminated polyesters, amine-terminated polyethers, and amine-terminated polyesters. Preferably, the polymer component is a hydroxy-terminated polyether.

Suitable hydroxy-terminated polyethers include alkoxylated derivatives of compounds comprising from two to five hydroxy groups, or mixtures thereof. For example, suitable polymer components include alkoxylated derivatives of ethylene glycol, propylene glycol, butane-1,4-diol, glycerol, trimethylolpropane, di- or polyethers- of trimethylolpropane, erythritol, pentaerythritol, pentane-1,2,4,5-tetrol, dextrose and/or sorbitol, preferably wherein the alkoxylated derivatives are ethoxylated derivates, propoxylated derivatives, or ethoxylated-co-propoxylated derivatives. Preferably, the hydroxy-terminated polyether may be an ethylene oxide capped propoxylated derivative of pentaerythritol. Ethylene oxide capping is preferred since low steric hindrance of the terminal hydroxyl groups promotes faster reaction with the isocyanate groups of the cross-linking component (B).

The polymer component (A) preferably has a weight average molecular weight in the range of 1,000 to 50,000 Dalton, preferably in the range of 1,000 to 20,000 Dalton, preferably in the range of 1,500 to 10,000 Dalton (as measured by GPC). Molecules within this range are generally understood to be incapable of being absorbed easily through the cell walls and hence are less cytotoxic. This is particularly important in medical applications where the adhesive composition or a medical product comprising the adhesive composition will be applied directly to skin. However, if the molecular weight of the polymer component (A) is too high, the adhesive will be insufficiently tacky.

The polymer component preferably has an equivalent weight per nucleophilic functional group of from 200 to 5,000, preferably from 500 to 2,000, preferably from 1,000 to 2,000.

The polymer component preferably has a mono-ol content of no more than 2 mol%, preferably no more than 1 mol%, preferably no more than 0.5 mol%. As described above, molecules of the polymer component which contain one nucleophilic functional group cannot contribute to effective cross-linking as they can only react with one free isocyanate group of the cross-linking component (B).

**Cross-linking component (B)**

The cross-linking component (B) of the adhesive polyurethane compositions of the invention is obtained by the reaction of a polyisocyanate component (i) with the at least one compound (ii) that comprises a functional group that is curable by free-radical polymerisation and further comprises a nucleophilic functional group containing an active hydrogen atom. The functional group that is curable by free-radical polymerisation provides the switching capability of the claimed adhesive compositions, and the nucleophilic functional group containing an active hydrogen atom enables attachment of the compound (ii) to the polyisocyanate component (i). The polyisocyanate component (i) is also reacted with at least one compound (compound (iii)) that comprises a nucleophilic functional group containing an active hydrogen atom but which does not include a functional group that is curable by free-radical polymerisation. Compound (iii) is therefore attached to the polyisocyanate component (i) but does not contribute to the switching capability of the claimed adhesive compositions.

The total degree of substitution of the polyisocyanate component (i) by the compounds (ii) and (iii) is no more than the lesser of 0.3Y and 0.8, and is preferably no more than the lesser of 0.28Y and 0.8, preferably no more than the lesser of 0.28Y and 0.75.

Although effective adhesives may be obtained even with very high degrees of substitution of the polyisocyanate component (i), such compositions will require a higher total amount of the polyisocyanate to ensure sufficient free isocyanate groups are still available for reaction with the polymer component (A). This may be undesirable for cost reasons.

The total degree of substitution of the polyisocyanate component (i) by the compounds (ii) and (iii) is at least 0.2, and is preferably at least 0.3, preferably at least 0.4. At very low degrees of substitution, the cross-linking component (B) comprises higher relative amounts of polyisocyanates that are either unsubstituted or monosubstituted. This results in tighter cross-linking of the polymer component, with more of the polymer end groups bound into nodal points with e.g. three or more polymer end groups attached. It has been found that this results in an adhesive having reduced peel strength. An improvement in peel strength is thus obtained by ensuring that the total degree of substitution of the polyisocyanate component (i) is at least 0.2, and preferably at least 0.3 or at least 0.4. Particularly good adhesive performance is found, for instance when the total degree of substitution of the polyisocyanate component (i) is at least 0.45, or at least 0.5.

For example, the total degree of substitution of the polyisocyanate component (i) by the compounds (ii) and (iii) may be at least 0.2 and no more than the lesser of 0.3Y and 0.8; at least 0.2 and no more than the lesser of 0.28Y and 0.8; at least 0.2 and no more than the lesser of 0.28Y and 0.75; at least 0.3 and no more than the lesser of 0.3Y and 0.8; at least 0.3 and no more than the lesser of 0.28Y and 0.8; at least 0.3 and no more than the lesser of 0.28Y and 0.75; at least 0.4 and no more than the lesser of 0.3Y and 0.8; at least 0.4 and no more than the lesser of 0.28Y and 0.8; at least 0.4 and no more than the lesser of 0.28Y and 0.75; at least 0.45 and no more than the lesser of 0.3Y and 0.8; at least 0.45 and no more than the lesser of 0.28Y and 0.8; at least 0.45 and no more than the lesser of 0.28Y and 0.75; at least 0.5 and no more than the lesser of 0.3Y and 0.8; at least 0.5 and no more than the lesser of 0.28Y and 0.8; or at least 0.5 and no more than the lesser of 0.28Y and 0.75.

As set out above, partial substitution of the polyisocyanate component (i) is desirable in order to obtain a distribution of different isocyanate-containing compounds in the cross-linking component (B). However, obtaining the required degree of substitution of the polyisocyanate component (i) with only the compound (ii) tends to result in an adhesive comprising significantly more curable groups than is necessary to obtain good switch performance. Although effective adhesives are obtained even with very high degrees of substitution of the polyisocyanate component (i) by the compound (ii), it is preferred that the adhesive composition does not comprise significantly more curable groups than are required to switch the adhesive. Excessive amounts of curable groups may necessitate careful handling during manufacture of the adhesive and the products that comprise the adhesive. Excessive substitution of the polyisocyanate component (i) by the compound (ii) is also disfavoured for cost, and biocompatibility reasons.

One particular consequence of compositions comprising a high concentration of curable groups is that shear forces in the apparatus (e.g. pumps and flow lines) used to handle adhesives during manufacturing processes may initiate premature curing of the curable groups. This may result in reduced tackiness of the adhesive product and in some cases cause blockage of the manufacturing apparatus. The use of the compound (iii) therefore allows these problems to be mitigated by enabling a sufficient degree of substitution of the polyisocyanate component (i) without introducing excessive curable groups into the adhesive composition.

The polyisocyanate component (i) is substituted with both the compound (ii) and the compound (iii), and the degree of substitution of the polyisocyanate component (i) by the compound (ii) may be no more than 0.6, preferably no more than 0.55, preferably no more than 0.5, preferably no more than 0.4, preferably no more than 0.3, preferably no more than 0.25, preferably no more than 0.2, preferably no more than 0.18.

The polyisocyanate component (i) is substituted with both the compound (ii) and the compound (iii), and the degree of substitution of the polyisocyanate component (i) by the compound (ii) is preferably at least 0.05, preferably at least 0.06, preferably at least 0.07, preferably at least 0.08, preferably at least 0.1, preferably at least 0.12 to ensure a sufficient content of curable groups for effective switching performance. At very low degrees of substitution of the polyisocyanate component (i) with the compound (ii), there is a lower relative amount of curable groups which may result in reduced switch performance of the adhesive upon curing.

The polyisocyanate component (i) is substituted by both the compound (ii) and the compound (iii), and the degree of substitution of the polyisocyanate component (i) by the compound (ii) may be: at least 0.05 and no more than 0.5; or at least 0.05 and no more than 0.4; or at least 0.05 and no more than 0.3; or at least 0.05 and no more than 0.25; or at least 0.05 and no more than 0.2; or at least 0.06 and no more than 0.4; or at least 0.06 and no more than 0.3; or at least 0.06 and no more than 0.25; or at least 0.06 and no more than 0.2; or at least 0.08 and no more than 0.4; or at least 0.08 and no more than 0.3; or at least 0.08 and no more than 0.25; or at least 0.08 and no more than 0.2; or at least 0.1 and no more than 0.3; or at least 0.1 and no more than 0.25; or at least 0.1 and no more than 0.2; or at least 0.1 and no more than 0.18; or at least 0.12 and no more than 0.3; or at least 0.12 and no more than 0.25; or at least 0.12 and no more than 0.2; or at least 0.12 and no more than 0.18.

**Polyisocyanate component (i)**

The polyisocyanate component (i) has an average of at least Y isocyanate functions per molecule, wherein Y represents a number which may be in the range from 1.8 to 6, preferably in the range from 2 to 4, preferably in the range from 2 to 3.6, preferably in the range from 2.1 to 3.5, preferably in the range from 2.2 to 3.4. It will be understood that the polyisocyanate component (i) may be a single polyisocyanate compound or may comprise a mixture of different polyisocyanate compounds, so long as the average number of polyisocyanate groups per molecule is within the specified ranges. The term "average" in this context refers to the mean average number of isocyanate functions per isocyanate-containing molecule (i.e. [total moles isocyanate functional groups]/[total moles isocyanate-containing molecules])

When Y is within these ranges, the polyisocyanate component (i) includes an appropriate mixture of substituted polyisocyanates having different degrees of substitution. It has been found that this results in improved adhesive properties.

The polyisocyanate component (i) may in principle be selected from any of the compounds comprising multiple isocyanate groups that are known in the art for the manufacture of polyisocyanates, as well as mixtures thereof. For example, the polyisocyanate component (i) may comprise one or more polyisocyanate compounds selected from the group of polyisocyanates containing from 2 to 10 isocyanate functions per molecule and mixtures thereof, so long as Y is within the range from 1.8 to 6. Preferably, the polyisocyanate component (i) comprises one or more polyisocyanate compounds selected from the group of polyisocyanates containing from 2 to 4 isocyanate functions per molecule.

Optionally, the polyisocyanate component (i) may comprise a mixture of a diisocyanate and at least one polyisocyanate having an average of at least 3 isocyanate functions per molecule. It has been observed that mixtures of diisocyanates with other polyisocyanates result in adhesives which are more resistant to humidity. This is beneficial for medical applications in which the adhesive must stick to the skin, which may secrete sweat.

Examples of suitable polyisocyanates include diisocyanates of the formula OCN-R-NCO, wherein R independently represents a straight chain, branched or cyclic alkylene group having from 2 to 15 carbon atoms or an arylene group having from 6 to 20 carbon atoms. Examples of diisocyanates include hexamethylene diisocyanate, isophorone diisocyanate, toluene 2,4-diisocyanate, 4,4'-methylenebis(phenyl isocyanate), and 4,4'-methylenebis(cyclohexyl isocyanate). Further suitable polyisocyanates include polymers (e.g. dimers, trimers, tetramers etc.) based on the diisocyanates.

A preferred polyisocyanate is a trimerized diisocyanate of the formula $D(R-NCO)_3$, wherein D represents a ring structure selected from isocyanurate and iminooxadiazindione and mixtures thereof, and each R independently represents a straight chain, branched or cyclic alkylene group having from 2 to 15 carbon atoms or an arylene group having from 6 to 20 carbon atoms. A particularly preferred polyisocyanate is trimerized hexamethylene diisocyanate.

**Compound (ii)**

The cross-linking component (B) is obtained by the reaction of the polyisocyanate component (i) with at least one compound (ii) comprising a functional group that is curable by free-radical polymerisation and further comprises a nucleophilic functional group containing an active hydrogen atom.

The term "nucleophilic functional group containing an active hydrogen atom" refers herein to functional groups that are capable of undergoing an addition reaction with an isocyanate group to form an adduct. For example, the nucleophilic functional group containing an active hydrogen atom may be -OH, -COOH -NH, -SH, etc. Preferably the nucleophilic functional group containing an active hydrogen atom is a hydroxyl group (-OH). Preferably, the nucleophilic functional group containing an active hydrogen atom is a hydroxyl group attached to a primary carbon atom (i.e. a group of the formula $-CH_2OH$). Nucleophilic hydroxyl groups can readily react with the isocyanate groups of the polyisocyanate component (i) and hydroxyl-containing compounds containing curable moieties are readily available in the art. Preferably, the at least one compound (ii) comprises a single nucleophilic functional group containing an active hydrogen atom.

The at least one compound (ii) preferably comprises an olefin moiety as the functional group that is curable by free-radical polymerisation. The at least one compound (ii) may be a single compound, or a mixture of different compounds that each comprise an olefin moiety as the functional group that is curable by free-radical polymerisation. Olefin moieties (e.g. acrylates and methacrylates) tend to have high yields in polymerisation reactions and are thus good curable groups for achieving effective switch performance via a free-radical initiated curing process.

Preferably, the at least one compound (ii) comprises an olefin moiety as the functional group that is curable by free-radical polymerisation and a hydroxyl group as the nucleophilic functional group containing an active hydrogen atom.

In the broadest sense, any unsaturated compounds that are curable by free radical polymerisation and further comprise a nucleophilic functional group containing an active hydrogen atom can be used as the compound (ii). Examples of suitable compounds include hydroxy-substituted acrylate esters and hydroxy-substituted methacrylate esters. Preferably, the at least one compound (ii) is selected from hydroxy substituted-$(C_2-C_{20})$alkyl methacrylate esters, hydroxy substituted-$(C_2-C_{20})$alkyl acrylate esters, polyalkoxylated monomethacrylate esters containing 2-10 ether functions, polyalkoxylated monoacrylate esters containing 2-10 ether functions, and mixtures thereof. More preferably, the at least one compound (ii) is selected from hydroxy substituted-$(C_2-C_6)$alkyl methacrylate esters.

For example, the compound (ii) may be selected from 2-hydroxyethyl methacrylate, 2-hydroxypropylmethacrylate, 2-hydroxyethyl acrylate, 2-hydroxypropylacrylate polypropylene glycol monomethacrylate, and polypropylene glycol monoacrylate. Preferred compounds (ii) are 2-hydroxypropyl methacrylate, hydroxyethyl methacrylate and mixtures thereof.

The at least one compound (ii) may be a single compound, or may be a mixture of compounds, each of which has a functional group that is curable by free-radical polymerisation and a nucleophilic functional group containing an active hydrogen atom. In the case that compound (ii) is a mixture of compounds, each compound preferably comprises the same type of curable functional group, more preferably each compound comprises an olefin group. For example, compound (ii) may be a mixture of 2-hydroxypropyl methacrylate with the isomeric hydroxyisopropyl methacrylate. Other examples of hydroxyl containing acrylic esters are hydroxyl $(CH_2)_n$ methacrylic esters where n is 4-8, hydroxyethyl methacrylate caprolactone ester (caprolactone 2-(methacryloyloxy)ethyl ester), 3-(acryloyloxy)-2-hydroxypropyl methacrylate and glycerol dimethacrylate.

**Compound (iii)**

The cross-linking component (B) is obtained by reacting the polyisocyanate component (i) with both the at least one compound (ii) and the at least one compound (iii). The at least one compound (iii) comprises a nucleophilic functional group containing an active hydrogen atom but does not include a functional group that is curable by free radical polymerisation. The at least one compound (iii) may be a single compound, or it may be a mixture of different compounds, none of which includes a functional group that is curable by free-radical polymerisation.

As described above, only a certain content of curable groups is required to achieve good switch performance. Thus, compound (iii) is included in the adhesive compositions of the invention in order to obtain the advantageous distribution of compounds having different numbers of isocyanate groups in the cross-linking component (B) without incorporating excessive amounts of the curable groups.

The at least one compound (iii) is preferably selected from $C_1$-$C_{30}$ aliphatic alcohols, preferably linear, branched or cyclic $C_1$-$C_{18}$ aliphatic alcohols, more preferably linear, branched or cyclic $C_1$-$C_{12}$ aliphatic alcohols. Preferably the at least one compound (iii) is selected from branched $C_3$-$C_{12}$ aliphatic alcohols, or from branched $C_6$-$C_{18}$ aliphatic alcohols. An example of a suitable compound is 2-ethyl-1-hexanol. Compounds of this type serve simply to occupy one or more isocyanate bonding sites so as to obtain the required distribution of reaction products from the reaction of the polyisocyanate component (i) with the compounds (ii) and (iii). One advantage of including compounds of this type is that the aliphatic carbon chain adds a degree of fattiness to the adhesive composition. This reduces the possibility of lower molecular weight components of the adhesive being more water soluble and thereby more easily penetrating the skin, resulting in a further reduction in the cytotoxicity of the adhesive. In addition, the aliphatic carbon chains act as a plasticiser in the adhesive, decreasing the interaction between the polymer chains. As a result, the adhesive becomes softer, less expensive and easier to produce.

The at least one compound (iii) also enables other functionalities to be incorporated into the adhesive composition, without affecting the initial peel force or switch performance. Molecules containing these functionalities would otherwise be mixed in to the adhesive polyurethane composition as small molecules. Thus, by adding them into the polyurethane network, the cytotoxicity of the adhesive is reduced. For example, the at least one compound (iii) may comprise or consist of one or more hydroxy-substituted photoinitiators.

Other examples of compounds which may be incorporated into the adhesive composition of the invention as compound (iii) include biocides and catalysts.

Typically, the at least one compound (iii) comprises a single nucleophilic group containing an active hydrogen atom. However, it is not excluded that the at least one compound (iii) may include more than one nucleophilic functional group containing an active hydrogen atom, in which case the compound (iii) may link together two polyisocyanate molecules.

**Adhesive compositions**

It will be understood that cross-linking within the polyurethane network of the polyisocyanate may be the result of the reaction of polyisocyanate components comprising at least three unreacted isocyanate groups and/or the reaction of polymer components comprising at least three nucleophilic functional groups containing an active hydrogen atom. To ensure a sufficient level of cross-linking in the polyurethane adhesive composition of the invention, it is preferred that the sum of X and Y is at least 4.5, preferably at least 5, preferably at least 5.5, preferably at least 6. Preferably the sum of X and Y is no more than 10, preferably no more than 9, preferably no more than 8.

The relative amounts of the polymer component (A) and the cross-linking component (B) may be defined as the molar ratio of free isocyanate groups in the cross-linking component (B) to the available nucleophilic groups from the polymer component (A).

A lower molar ratio for a specific substitution degree yields a tackier but less cohesively strong adhesive. Accordingly, the skilled person can easily tune the ratio of the cross-linking component (B) to the polymer component (A) in order to obtain an adhesive with the required balance of adhesiveness vs cohesiveness.

For example, the molar ratio of unsubstituted isocyanate functions in the cross-linking component (B) to nucleophilic groups in the polymer component (A) will generally be least 0.3, preferably at least 0.4, preferably at least 0.5. Preferably, the molar ratio of unsubstituted isocyanate functions in the cross-linking component (B) to nucleophilic groups in the polymer component (A) is less than 1, preferably no more than 0.8, preferably no more than 0.7. For example the molar ratio of unsubstituted isocyanate functions in the cross-linking component (B) to nucleophilic groups in the polymer component (A) may be from 0.3 to 0.8, preferably from 0.4 to 0.75, preferably from 0.5 to 0.7.

The ratio of unsubstituted isocyanate functions in the cross-linking component (B) to nucleophilic groups in the polymer component (A) is preferably at least 0.8n and no more than the lesser of 1.2n and 0.8, wherein n represents the total degree of substitution of the polyisocyanate component (i). It is found that controlling the ratio within these ranges provides an optimum balance of tackiness and cohesiveness of the adhesive at different degrees of substitution of the polyisocyanate component (i).

The adhesive polyurethane composition of the invention preferably comprises from 0.05 to 1 meq/g, preferably from 0.06 to 0.5 meq/g, preferably from 0.08 to 0.4 meq/g, preferably from 0.1 to 0.3 meq/g, preferably from 0.12 to 0.25 meq/g, preferably from 0.15 to 0.2 meq/g of the functional group that is curable by free-radical polymerisation.

**Photoinitiators**

The adhesive polyurethane composition of the invention preferably comprises a photoinitiator, preferably from 0.05 to 5 wt% of a photoinitiator, preferably from 0.1 to 5 wt% of a photoinitiator preferably from 0.2 to 2 wt% of a photoinitiator. The photoinitiator may be mixed in to the adhesive composition and/or bound to the polymeric chains of the adhesive composition.

The photoinitiator may be any species which is capable of producing radical species under mild conditions, e.g. UV or visible light, in order to promote free-radical initiated polymerization of the curable functional groups of compound (ii).

Preferably, the photoinitiator is reactive to UV radiation having wavelength in the range from 200 to 400 nm), preferably UVA radiation (315 to 400 nm). UVA is particularly preferred for medical applications and other applications requiring exposure of humans or animals to the UV radiation. The range of 200 to 400 nm is referred to herein as "long wavelength UV".

The photoinitiator may alternatively produce radical species upon exposure to visible light, but products that are curable upon exposure to visible light require careful handling and/or require additional visible light occlusive materials to be incorporated in the product to avoid premature switching of the adhesive. The visible light occlusive material needs to be removed from the product at the appropriate time, when switching is desired.

The photoinitiator reactive to UV may be selected from any of the conventional photoinitiators known in the art. For example, the photoinitiator reactive to UV may suitably be selected from the group consisting of benzoin and derivatives (e.g the ethyl, isopropyl or isobutyl ethers of benzoin); benzophenone and derivatives (e.g. 4-phenylbenzophenone); acetophenone and 4'-phenoxyacetophenone; 2-methyl-1-[4-(methylthio)phenyl]-2-(4-morpholinyl)-1-propanone, 2-benzyl-2-(dimethylamino)-1-[4-(4-morpholinyl)phenyl]-1-butanone; 2-dimethylamino-2-(4-methyl-benzyl)-1-(4-morpholin-4-yl-phenyl)-butan-1-one; 2-ethyl anthraquinone; benzil dimethyl ketal; 2-hydroxy-2-methyl propiophenone; ethyl-4-(dimethylamino) benzoate.

Suitable free radical initiators for visible light activation include titanocene photoinitiators; dye/co-initiator systems, e.g., thionine/triethanol-amine; dye/peroxide systems and 1,2-diketone/co-initiator systems, e.g., camphor-quinone/tertiary amine. Examples of visible light photoinitiators are: phenanthrenequinone; titanocenes; and bis(2,4,6-trimethyl-benzoyl)-phenylphosphineoxide.

The adhesive polyurethane composition preferably comprises from 0.05 to 5 wt% of a photoinitiator, preferably from 0.2 to 2 wt% of a photoinitiator. These particular ranges of photoinitiator enable the curable groups to react with one another upon activation, and allow the adhesive to achieve desirable switch times.

**Solvent**

The adhesive polyurethane composition may further comprise a solvent. The solvent must be a non-protic solvent so that it does not react with the isocyanate groups of the polyisocyanate component (i). Preferably, the solvent has low toxicity, preferably the solvent is non-toxic. An example of a preferred solvent is ethyl acetate.

**Stabiliser**

The switchable adhesive composition may also contain a stabiliser. As used herein, the term "stabiliser" refers to a substance that is added to the adhesive composition to scavenge free-radicals so as to prevent premature reaction of the curable functional groups in the adhesive during manufacture and/or storage of the adhesive composition. These substances are well-known in the art of curable materials and may also be referred to as anti-oxidants.

Examples of suitable stabilisers include 1-piperidinyloxy-4,4'-[1,10-dioxo-1,10-decanediyl)bis(oxy)]bis[2,2,6,6-tetramethyl] and phenolic derivatives such as methoxyphenol, di-tert-butyl-4-methylphenol, and pentaerythritol tetrakis(3-(3,5-di-tert-butyl-4-hydroxy-phenyl)propionate).

**Optional Constituents**

The switchable adhesive composition may also include photo-sensitisers. Since a sensitising species often absorbs energy in a different part of the spectrum from the initiator, more effective use of the light source may be achievable through the incorporation of sensitisers into the composition. Many photo-sensitisers are complex organic molecules, absorbing in the long wavelength UV and/or visible portion of the spectrum.

The adhesive polyurethane composition may also incorporate scattering particles to increase the effect of irradiation of the adhesive mixture by scattering the irradiating UV or visible light through the thickness of the adhesive mixture. Preferably, the light scattering particles are an inorganic compound such as silica powder, alumina powder, silica-alumina powder or mica powder with particle sizes of the order of 10 nm or greater, typically up to 1 $\mu$m.

The adhesive polyurethane composition may also include a component containing aliphatic thiol groups for reducing oxygen inhibition of the radical polymerization at the surface and preventing the surface from remaining tacky after cure when the surface is not protected by a film (second release liner). Apart from having oxygen-scavenging properties the aliphatic thiols can also take part in the radical polymerization of the curable molecules via thiol-ene reactions. For more effective contribution to the curing reaction a component with two or more thiol groups could be used, such as trimethylolpropanetris(3-mercaptopropionate) or pentaerythritoltetrakis(2-mercaptoacetate). Amine synergists such as triethanol amine, ethyl-4-dimethylaminobenzoate or acrylated amines could also be used to reduce oxygen inhibition of the radical polymerization at the adhesive surface when this is not protected by a film.

The reactivity of the composition can be increased by increasing the concentration (meq/g) of curable groups in the adhesive, by using a compound (ii) with two or more curable groups, and/or by using more reactive functionalities in the adhesive, e.g., partly or completely exchanging methacrylate with acrylate (acrylates are more reactive but also slightly more toxic) .

The interactions between molecules of the adhesive and thereby the viscosity of the adhesive can be decreased by using bulky groups (e.g., by adding methyl groups or by branching of the molecular chain) and/or introducing asymmetry or higher hydrophobicity in the cross-linking component (B). This may be achieved by using polyisocyanate components (i) which have bulky groups e.g., by exchanging hexamethylene diisocyanate with trimethylhexamethylene diisocyanate, exchanging isocyanurate with iminooxadiazinedione, exchanging butanediol with methylpentandiol, exchanging polyethylene glycol with polypropylene glycol, etc. Alternatively, this may also be achieved by using at least one compound (iii) in the cross-linking component (B), wherein the at least one compound (iii) comprises bulky groups.

The adhesive polyurethane compositions of the invention exhibit a reduction in peel force of after switching of at least 30%, and preferably from 50 to 99%, preferably from 70 to 99% when measured according to the method described below.

In accordance with the first aspect of the invention, there are further provided adhesive polyurethane compositions according to the following aspects 1-1 to 1-18.

Aspect 1-1: An adhesive polyurethane composition according to the first aspect of the invention, wherein:

(i) X represents a number having a value of at least 2.2;
(ii) Y represents a number in the range from 2 to 4; and
(iii) the total degree of substitution of the polyisocyanate component (i) is at least 0.3 and no more than the lesser of 0.28Y and 0.75; and
(iv) the molar ratio of unsubstituted isocyanate functions in the cross-linking component (B) to nucleophilic groups in the polymer component (A) is from 0.3 to 0.8.

Aspect 1-2: An adhesive polyurethane composition according to the first aspect of the invention, wherein:

(i) X represents a number having a value of at least 2.6;
(ii) Y represents a number in the range from 2.1 to 3.5; and
(iii) the total degree of substitution of the polyisocyanate component (i) is at least 0.4 and no more than the lesser of 0.28Y and 0.75; and
(iv) the molar ratio of unsubstituted isocyanate functions in the cross-linking component (B) to nucleophilic groups in the polymer component (A) is from 0.4 to 0.75.

Aspect 1-3: An adhesive polyurethane composition according to the first aspect of the invention, wherein:

(i) X represents a number having a value of at least 2.8;
(ii) Y represents a number in the range from 2.2 to 3.4; and
(iii) the total degree of substitution of the polyisocyanate component (i) is at least 0.5 and no more than the lesser of 0.28Y and 0.75; and
(iv) the molar ratio of unsubstituted isocyanate functions in the cross-linking component (B) to nucleophilic groups in the polymer component (A) is from 0.5 to 0.7.

Aspect 1-4: An adhesive polyurethane composition according to the first aspect of the invention, wherein:

(i) X represents a number having a value of at least 2.2;
(ii) Y represents a number in the range from 2 to 4; and
(iii) the total degree of substitution of the polyisocyanate component (i) is at least 0.3 and no more than the lesser of 0.28Y and 0.75;
(iv) the molar ratio of unsubstituted isocyanate functions in the cross-linking component (B) to nucleophilic groups in the polymer component (A) is from 0.3 to 0.8;
(v) the cross-linking component (B) is obtained by reacting the polyisocyanate component (i) with both the compound (ii) and the compound (iii); and
(vi) the degree of substitution of the polyisocyanate component (i) by the compound (ii) is from 0.05 to 0.5.

Aspect 1-5: An adhesive polyurethane composition according to the first aspect of the invention, wherein:

(i) X represents a number having a value of at least 2.6;
(ii) Y represents a number in the range from 2.1 to 3.5; and
(iii) the total degree of substitution of the polyisocyanate component (i) is at least 0.4 and no more than the lesser of 0.28Y and 0.75;
(iv) the molar ratio of unsubstituted isocyanate functions in the cross-linking component (B) to nucleophilic groups in the polymer component (A) is from 0.4 to 0.75;
(v) the cross-linking component (B) is obtained by reacting the polyisocyanate component (i) with both the compound (ii) and the compound (iii); and
(vi) the degree of substitution of the polyisocyanate component (i) by the compound (ii) is from 0.08 to 0.4.

Aspect 1-6: An adhesive polyurethane composition according to the first aspect of the invention, wherein:

(i) X represents a number having a value of at least 2.8;
(ii) Y represents a number in the range from 2.2 to 3.4; and
(iii) the total degree of substitution of the polyisocyanate component (i) is at least 0.5 and no more than the lesser of 0.28Y and 0.75;
(iv) the molar ratio of unsubstituted isocyanate functions in the cross-linking component (B) to nucleophilic groups in the polymer component (A) is from 0.5 to 0.7;
(v) the cross-linking component (B) is obtained by reacting the polyisocyanate component (i) with both the compound (ii) and the compound (iii); and
(vi) the degree of substitution of the polyisocyanate component (i) by the compound (ii) is from 0.1 to 0.3.

Aspect 1-7: An adhesive polyurethane composition according to the first aspect of the invention, wherein:

(i) X represents a number having a value of at least 2.2;
(ii) Y represents a number in the range from 2 to 4; and
(iii) the total degree of substitution of the polyisocyanate component (i) is at least 0.3 and no more than the lesser of 0.28Y and 0.75;
(iv) the molar ratio of unsubstituted isocyanate functions in the cross-linking component (B) to nucleophilic groups in the polymer component (A) is from 0.3 to 0.8;
(v) the polymer component (A) is selected from hydroxy-terminated polyethers and hydroxy-terminated polyesters having a weight average molecular weight in the range from 1,000 to 20,000 Dalton; and
(vi) the at least one compound (ii) is selected from hydroxy substituted-$(C_2-C_{20})$alkyl methacrylate esters and hydroxy substituted-$(C_2-C_{20})$alkyl acrylate esters.

Aspect 1-8: An adhesive polyurethane composition according to the first aspect of the invention, wherein:

(i) X represents a number having a value of at least 2.6;

(ii) Y represents a number in the range from 2.1 to 3.5; and

(iii) the total degree of substitution of the polyisocyanate component (i) is at least 0.4 and no more than the lesser of 0.28Y and 0.75;

(iv) the molar ratio of unsubstituted isocyanate functions in the cross-linking component (B) to nucleophilic groups in the polymer component (A) is from 0.4 to 0.75;

(v) the polymer component (A) is selected from hydroxy-terminated polyethers and hydroxy-terminated polyesters having a weight average molecular weight in the range from 1,000 to 20,000 Dalton; and

(vi) the at least one compound (ii) is selected from hydroxy substituted-$(C_2-C_{20})$alkyl methacrylate esters and hydroxy substituted-$(C_2-C_{20})$alkyl acrylate esters.

Aspect 1-9: An adhesive polyurethane composition according to the first aspect of the invention, wherein:

(i) X represents a number having a value of at least 2.8;

(ii) Y represents a number in the range from 2.2 to 3.4; and

(iii) the total degree of substitution of the polyisocyanate component (i) is at least 0.5 and no more than the lesser of 0.28Y and 0.75;

(iv) the molar ratio of unsubstituted isocyanate functions in the cross-linking component (B) to nucleophilic groups in the polymer component (A) is from 0.5 to 0.7;

(v) the polymer component (A) is selected from hydroxy-terminated polyethers and hydroxy-terminated polyesters having a weight average molecular weight in the range from 1,000 to 20,000 Dalton; and

(vi) the at least one compound (ii) is selected from hydroxy substituted-$(C_2-C_{20})$alkyl methacrylate esters and hydroxy substituted-$(C_2-C_{20})$alkyl acrylate esters.

Aspect 1-10: An adhesive polyurethane composition according to the first aspect of the invention, wherein:

(i) X represents a number having a value of at least 2.2;

(ii) Y represents a number in the range from 2 to 4; and

(iii) the total degree of substitution of the polyisocyanate component (i) is at least 0.3 and no more than the lesser of 0.28Y and 0.75;

(iv) the molar ratio of unsubstituted isocyanate functions in the cross-linking component (B) to nucleophilic groups in the polymer component (A) is from 0.3 to 0.8;

(v) the cross-linking component (B) is obtained by reacting the polyisocyanate component (i) with both the compound (ii) and the compound (iii);

(vi) the degree of substitution of the polyisocyanate component (i) by the compound (ii) is from 0.05 to 0.5;

(vii) the polymer component (A) is selected from hydroxy-terminated polyethers and hydroxy-terminated polyesters having a weight average molecular weight in the range from 1,000 to 20,000 Dalton;

(viii) the at least one compound (ii) is selected from hydroxy substituted-$(C_2-C_{20})$alkyl methacrylate esters and hydroxy substituted-$(C_2-C_{20})$alkyl acrylate esters; and

(ix) the at least one compound (iii) is selected from $C_1-C_{30}$ aliphatic alcohols.

Aspect 1-11: An adhesive polyurethane composition according to the first aspect of the invention, wherein:

(i) X represents a number having a value of at least 2.6;

(ii) Y represents a number in the range from 2.1 to 3.5; and

(iii) the total degree of substitution of the polyisocyanate component (i) is at least 0.4 and no more than the lesser of 0.28Y and 0.75;

(iv) the molar ratio of unsubstituted isocyanate functions in the cross-linking component (B) to nucleophilic groups in the polymer component (A) is from 0.4 to 0.75;

(v) the cross-linking component (B) is obtained by reacting the polyisocyanate component (i) with both the compound (ii) and the compound (iii);

(vi) the degree of substitution of the polyisocyanate component (i) by the compound (ii) is from 0.08 to 0.4;

(vii) the polymer component (A) is selected from hydroxy-terminated polyethers and hydroxy-terminated polyesters having a weight average molecular weight in the range from 1,000 to 20,000 Dalton;

(viii) the at least one compound (ii) is selected from hydroxy substituted-$(C_2-C_{20})$alkyl methacrylate esters and hydroxy substituted-$(C_2-C_{20})$alkyl acrylate esters; and

(ix) the at least one compound (iii) is selected from $C_1-C_{30}$ aliphatic alcohols.

Aspect 1-12: An adhesive polyurethane composition according to the first aspect of the invention, wherein:

(i) X represents a number having a value of at least 2.8;
(ii) Y represents a number in the range from 2.2 to 3.4; and
(iii) the total degree of substitution of the polyisocyanate component (i) is at least 0.5 and no more than the lesser of 0.28Y and 0.75;
(iv) the molar ratio of unsubstituted isocyanate functions in the cross-linking component (B) to nucleophilic groups in the polymer component (A) is from 0.5 to 0.7;
(v) the cross-linking component (B) is obtained by reacting the polyisocyanate component (i) with both the compound (ii) and the compound (iii); and
(vi) the degree of substitution of the polyisocyanate component (i) by the compound (ii) is from 0.1 to 0.3.;
(vii) the polymer component (A) is selected from hydroxy-terminated polyethers and hydroxy-terminated polyesters having a weight average molecular weight in the range from 1,000 to 20,000 Dalton;
(viii) the at least one compound (ii) is selected from hydroxy substituted-($C_2$-$C_{20}$)alkyl methacrylate esters and hydroxy substituted-($C_2$-$C_{20}$)alkyl acrylate esters; and
(ix) the at least one compound (iii) is selected from $C_1$-$C_{30}$ aliphatic alcohols.

Aspect 1-13: An adhesive polyurethane composition according to the first aspect of the invention, wherein:

(i) X represents a number having a value of at least 2.2;
(ii) Y represents a number in the range from 2 to 4; and
(iii) the total degree of substitution of the polyisocyanate component (i) is at least 0.3 and no more than the lesser of 0.28Y and 0.75;
(iv) the molar ratio of unsubstituted isocyanate functions in the cross-linking component (B) to nucleophilic groups in the polymer component (A) is from 0.3 to 0.8;
(v) the polymer component (A) is selected from hydroxy-terminated polyethers and hydroxy-terminated polyesters having a weight average molecular weight in the range from 1,000 to 20,000 Dalton; and
(vi) the at least one compound (ii) is selected from hydroxy substituted-($C_2$-$C_6$)alkyl methacrylate esters.

Aspect 1-14: An adhesive polyurethane composition according to the first aspect of the invention, wherein:

(i) X represents a number having a value of at least 2.6;
(ii) Y represents a number in the range from 2.1 to 3.5; and
(iii) the total degree of substitution of the polyisocyanate component (i) is at least 0.4 and no more than the lesser of 0.28Y and 0.75;
(iv) the molar ratio of unsubstituted isocyanate functions in the cross-linking component (B) to nucleophilic groups in the polymer component (A) is from 0.4 to 0.75;
(v) the polymer component (A) is selected from hydroxy-terminated polyethers and hydroxy-terminated polyesters having a weight average molecular weight in the range from 1,000 to 20,000 Dalton; and
(vi) the at least one compound (ii) is selected from hydroxy substituted-($C_2$-$C_6$)alkyl methacrylate esters.

Aspect 1-15: An adhesive polyurethane composition according to the first aspect of the invention, wherein:

(i) X represents a number having a value of at least 2.8;
(ii) Y represents a number in the range from 2.2 to 3.4; and
(iii) the total degree of substitution of the polyisocyanate component (i) is at least 0.5 and no more than the lesser of 0.28Y and 0.75;
(iv) the molar ratio of unsubstituted isocyanate functions in the cross-linking component (B) to nucleophilic groups in the polymer component (A) is from 0.5 to 0.7;
(v) the polymer component (A) is selected from hydroxy-terminated polyethers and hydroxy-terminated polyesters having a weight average molecular weight in the range from 1,000 to 20,000 Dalton; and
(vi) the at least one compound (ii) is selected from hydroxy substituted-($C_2$-$C_6$)alkyl methacrylate esters.

Aspect 1-16: An adhesive polyurethane composition according to the first aspect of the invention, wherein:

(i) X represents a number having a value of at least 2.2;
(ii) Y represents a number in the range from 2 to 4; and
(iii) the total degree of substitution of the polyisocyanate component (i) is at least 0.3 and no more than the lesser of 0.28Y and 0.75;

(iv) the molar ratio of unsubstituted isocyanate functions in the cross-linking component (B) to nucleophilic groups in the polymer component (A) is from 0.3 to 0.8;

(v) the cross-linking component (B) is obtained by reacting the polyisocyanate component (i) with both the compound (ii) and the compound (iii);

(vi) the degree of substitution of the polyisocyanate component (i) by the compound (ii) is from 0.05 to 0.5;

(vii) the polymer component (A) is selected from hydroxy-terminated polyethers and hydroxy-terminated polyesters having a weight average molecular weight in the range from 1,000 to 20,000 Dalton;

(viii) the at least one compound (ii) is selected from hydroxy substituted-$(C_2\text{-}C_6)$alkyl methacrylate esters; and

(ix) the at least one compound (iii) is selected from branched $C_3\text{-}C_{12}$ aliphatic alcohols.

Aspect 1-17: An adhesive polyurethane composition according to the first aspect of the invention, wherein:

(i) X represents a number having a value of at least 2.6;

(ii) Y represents a number in the range from 2.1 to 3.5; and

(iii) the total degree of substitution of the polyisocyanate component (i) is at least 0.4 and no more than the lesser of 0.28Y and 0.75;

(iv) the molar ratio of unsubstituted isocyanate functions in the cross-linking component (B) to nucleophilic groups in the polymer component (A) is from 0.4 to 0.75;

(v) the cross-linking component (B) is obtained by reacting the polyisocyanate component (i) with both the compound (ii) and the compound (iii);

(vi) the degree of substitution of the polyisocyanate component (i) by the compound (ii) is from 0.08 to 0.4;

(vii) the polymer component (A) is selected from hydroxy-terminated polyethers and hydroxy-terminated polyesters having a weight average molecular weight in the range from 1,000 to 20,000 Dalton;

(viii) the at least one compound (ii) is selected from hydroxy substituted-$(C_2\text{-}C_6)$alkyl methacrylate esters; and

(ix) the at least one compound (iii) is selected from branched $C_3\text{-}C_{12}$ aliphatic alcohols.

Aspect 1-18: An adhesive polyurethane composition according to the first aspect of the invention, wherein:

(i) X represents a number having a value of at least 2.8;

(ii) Y represents a number in the range from 2.2 to 3.4; and

(iii) the total degree of substitution of the polyisocyanate component (i) is at least 0.5 and no more than the lesser of 0.28Y and 0.75;

(iv) the molar ratio of unsubstituted isocyanate functions in the cross-linking component (B) to nucleophilic groups in the polymer component (A) is from 0.5 to 0.7;

(v) the cross-linking component (B) is obtained by reacting the polyisocyanate component (i) with both the compound (ii) and the compound (iii); and

(vi) the degree of substitution of the polyisocyanate component (i) by the compound (ii) is from 0.1 to 0.3.;

(vii) the polymer component (A) is selected from hydroxy-terminated polyethers and hydroxy-terminated polyesters having a weight average molecular weight in the range from 1,000 to 20,000 Dalton;

(viii) the at least one compound (ii) is selected from hydroxy substituted-$(C_2\text{-}C_6)$alkyl methacrylate esters; and

(ix) the at least one compound (iii) is selected from branched $C_3\text{-}C_{12}$ aliphatic alcohols.

It is to be understood that any preferred/optional features disclosed herein in relation to the first aspect of the invention that fall within the scope of the above-described aspects 1-1 to 1-18 are also preferred/optional features of the aspects 1-1 to 1-18. Likewise, any features of the dependent claims that fall within the scope of the above-described aspects 1-1 to 1-18 are also to be interpreted as though those claims also depended from aspects 1-1 to 1-18.

**Method of Preparing an Adhesive Polyurethane Composition**

According to a second aspect of the invention, there is provided a method of preparing an adhesive polyurethane composition comprising:

(a) a first step of reacting:

(i) a polyisocyanate component having an average of at least Y isocyanate functions per molecule, wherein Y represents a number in the range from 1.8 to 6; with

(ii) at least one compound comprising a functional group that is curable by free-radical polymerisation and further comprising a nucleophilic functional group containing an active hydrogen atom; and with (ii) at least one compound comprising a nucleophilic functional group containing an active hydrogen atom and which does not include a functional group that is curable by free-radical polymerisation;

wherein the total degree of substitution of the polyisocyanate component (i) by the compounds (ii) and (iii) is at least 0.2 and no more than the lesser of 0.3Y and 0.8, to form a cross-linking component (B);
(b) a second step of combining the product of step (a) with a polymer component (A) having a weight average molecular weight in the range of 500 to 100,000 Dalton, and containing an average per molecule of X nucleophilic functional groups containing an active hydrogen atom, wherein X represents a number greater than 2.

The method of the second aspect of the invention may be used to produce the adhesive polyurethane composition of the first aspect of the invention. Accordingly, any feature described as optional or preferred with reference to the first aspect of the invention shall also be understood to constitute an optional or preferred feature with respect to the identity, amounts and ratios of the corresponding components used in the method of the second aspect of the invention. For example, the method of the second aspect of the invention may be used to produce the adhesive polyurethane composition of any of aspects 1-1 to 1-18 described above.

Step (a) and/or step (b) of the method of preparing an adhesive polyurethane composition may be carried out in the presence of a catalyst. Suitable catalysts include dibutyltin dilaurate, Zirconium (IV) acetylacetonate, dibutyltin 2-ethylhexanoate, and tertiary amines.

Step (a) and/or step (b) may be carried out in the presence of a solvent. Suitable solvents are non-protic solvents, such as ethyl acetate, toluene and tetrahydrofuran.

Preferably, a photoinitiator is combined with the product of step (a) or with the polymer component prior to step (b). Most preferably, the photoinitiator is combined with the polymer component prior to step (b).

## Adhesive Medical Products

According to a third aspect of the invention, there is provided an adhesive medical product comprising a layer of a switchable adhesive polyurethane composition as defined herein, disposed between a first carrier film and a release liner.

Said adhesive medical product may comprise a product selected from the group consisting of an adhesive dressing including an absorbent wound pad, a surgical incision drape, a bacterial barrier for covering a wound, and a skin closure device for closing together the edges of a wound. Suitably, the first carrier film of the medical product may be translucent to UV and/or visible light. Optionally a removable light occlusive layer is laminated to the first carrier film on the surface opposite the adhesive composition.

Exemplary materials for the carrier film for carrying the switchable adhesive composition layer include polyethylene, polypropylene, polyurethane, ethylene/propylene copolymers, ethylene/ethylacrylate copolymers, ethylene/vinyl acetate copolymers, silicone elastomers, polydimethylsiloxanes, neoprene rubber, polyisobutylene, polyacrylates, chlorinated poly-ethylene, polyvinyl chloride, vinyl chloride-vinyl acetate copolymer, crosslinked polymethacrylate polymers (hydrogel), polyvinylidene chloride, poly (ethylene terephthalate), butyl rubber, epichlorohydrin rubbers, ethylenevinyl alcohol copolymers, ethylene- vinyloxyethanol copolymers; silicone copolymers, for example, polysiloxane- polycarbonate copolymers, polysiloxanepolyethylene oxide copolymers, polysiloxane-polymethacrylate copolymers, polysiloxane-alkylene copolymers (e.g., polysiloxane-ethylene copolymers), polysiloxane-alkylenesilane copolymers (e.g., polysiloxane-ethylenesilane copolymers), and the like; cellulose polymers, for example methyl or ethyl cellulose, hydroxy propyl methyl cellulose, and cellulose esters; polycarbonates; polytetrafluoro-ethylene; and the like. More preferred are medical grade polyethers or polyester polyurethanes, thermoplastic polyester elastomer, perforated polyethylene, polypropylene and PET films, as well as medical grade woven or non-woven materials.

Adhesive wound dressings usually consist of an absorbent pad for absorbing exudates from the dressed wound, the absorbent pad being surrounded by an adhesive area for securing the wound pad in position over the wound. The adhesive area and the wound pad are supported on a carrier film which is often flesh coloured or which may sometimes have an attractive design on its visible surface. The switchable adhesive compositions according to the invention are ideal candidates for use as the adhesive in the adhesive area around the wound pad of an adhesive wound dressing.

Adhesive wound dressings are often applied by lay users in their own homes and are not necessarily apply by medical practitioners. Since few homes have access to suitable UV irradiation equipment, adhesive wound dressings for home use preferably include an adhesive that is curable by visible light. In some cases it is preferable that adhesive wound dressings include an adhesive that is curable by UV light so that the timing of removal of the dressing is controlled by a medical practitioner. In the case that the adhesive is curable by UV light, a removable light occlusive layer may not be necessary.

Adhesive wound dressings intended for domestic use will preferably include light occlusive layers to prevent premature switching of the switchable adhesive composition. The first light occlusive layer is positioned on the opposite side of the carrier film to the adhesive and remains in place until such time as the user wants to remove the adhesive wound dressing. At this point, the first light occlusive layer is removed, exposing the underlying switchable adhesive composition to visible light, resulting in switching of the adhesive composition from a tacky state to a non-tacky or low-tack state. Optionally, a second light occlusive layer may form part of the release liner that is placed on the adhesive side of the dressing. This second light occlusive layer is removed just before the adhesive wound dressing is applied over a wound. The second light occlusive layer may be unnecessary in the case that the adhesive wound dressing is provided in a light occlusive package.

In the case that the adhesive wound dressing includes an adhesive that is curable by UV light, light occlusive layers are not necessary. Instead, the carrier film of the adhesive wound dressing only needs to be translucent to UV. When it is desired to remove the adhesive wound dressing, a trained medical practitioner shines a suitable UV light source over the adhesive wound dressing to initiate the curing reaction. Within seconds the adhesive loses its tackiness and the wound dressing is easily removed.

The release liner may be selected from any covering material having low surface energy to enable it to be removed easily from the adhesive layer. Suitable release liners include paper and plastic films provided with silicone coatings on the surface that contacts the adhesive composition.

Also disclosed herein, but not part of the claimed invention, is a method of treating a wound using a switchable adhesive dressing as defined herein, wherein the method comprises removing the release liner and applying the dressing to the wound site. The invention will be further described by way of example only and without limitation by reference to the drawings in which:

Figure 1 is a cross-sectional view through an adhesive dressing in accordance with a first embodiment of the invention;

Figure 2 is a perspective view showing the attempted removal from a patient's forearm of an adhesive dressing in accordance with the first embodiment of the invention and includes an enlargement bubble showing in partial cross-section how removal of the adhesive dressing causes the adhesive composition to extrude;

Figure 3 is a perspective view showing the adhesive dressing in accordance with the first embodiment of the invention undergoing irradiation to effect switching of the adhesive;

Figure 4 is a perspective view showing how the adhesive dressing in accordance with the first embodiment of the invention may be easily removed after switching of the adhesive;

Figure 5 is a graph showing the peel force measurements for examples 7 to 11.

## Detailed Description of the Drawings

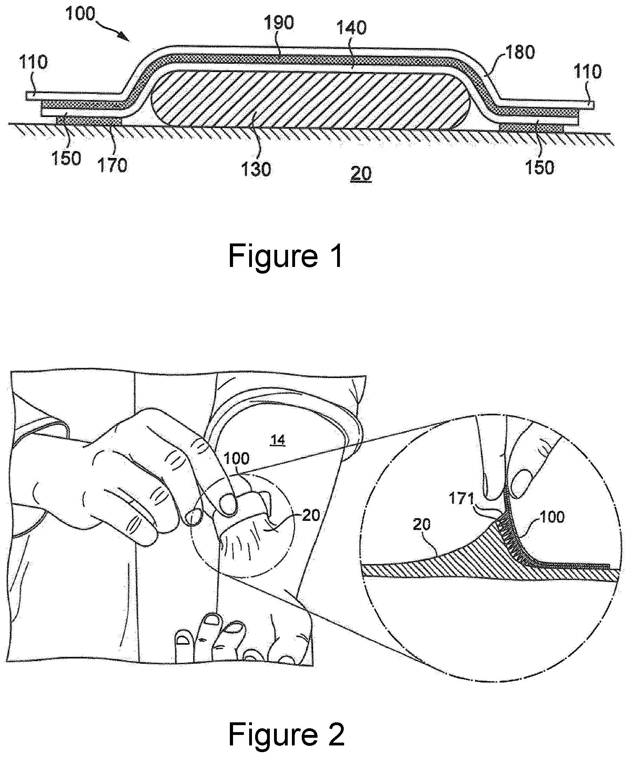

An adhesive medical product using the switchable adhesive composition of the present invention will now be described with reference to Figures 1 to 4. The adhesive medical product in this example is an adhesive medical dressing.

Figure 1 is a cross-sectional view through an adhesive medical dressing 100 attached to a patient's skin 20. The adhesive medical dressing 100 is a multi-layer product having the following structure. The dressing 100 comprises a wound facing absorbent layer 130 disposed beneath a protective backing layer 140. At opposed edges 150, the backing layer 140 is provided with a switchable adhesive composition 170 which includes curable molecules that can be cross-linked under the influence of UV and/or visible light.

The backing layer 140 is optionally provided with a light occlusive cover layer 180 which is releasably secured to the backing layer 140 by a weak adhesive 190. For ease of removal, the light occlusive cover layer 180 overlaps the backing layer 140 at its edges 110. In the case that the switchable adhesive composition 170 contains a photoinitiator that is actuated by UV radiation, the light occlusive cover layer 180 may be omitted.

Figure 2 is a perspective view showing the attempted removal of an adhesive dressing 100 from the forearm 14 of a patient, prior to switching of the switchable adhesive composition. Before switching, the adhesive composition 171 is very tacky and sticks the adhesive dressing 100 to the patient's skin 20 quite firmly. Hence, when the patient attempts to peel the dressing 100 from the forearm 14, the the dressing 100 remains attached to the skin 20 unless the dressing is peeled with some force.

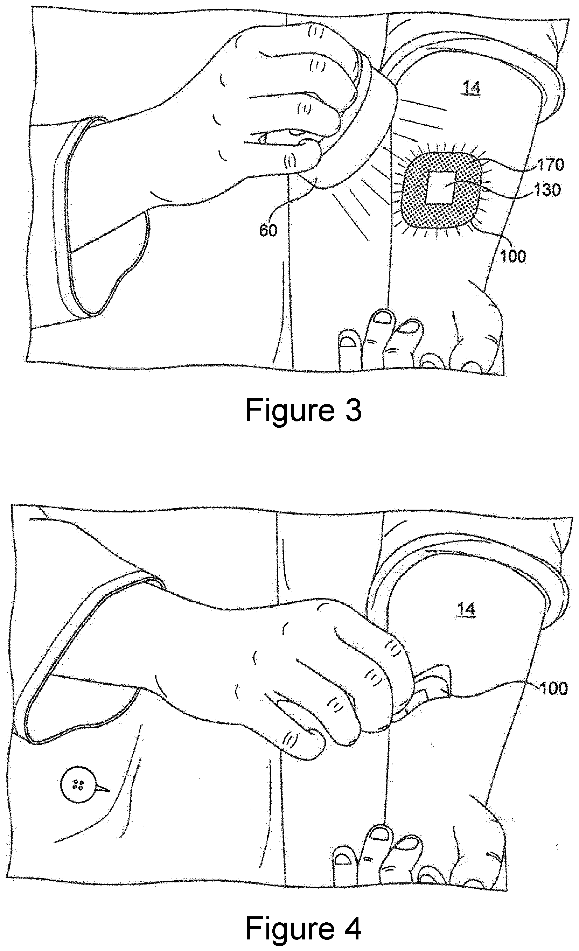

Figure 3 is a perspective view showing the adhesive dressing undergoing irradiation, in this example from a lamp 60, to effect cure of the curable molecules in the adhesive composition 170. The light from the lamp 60 (UV light or visible light, preferably long wavelength UV) causes the photoinitiator in the adhesive composition 170 to generate free radicals that initiate curing of the curable molecules in the adhesive composition. Curing transforms (switches) the adhesive composition 170 from its tacky state to a non-tacky or low-tack state.

Figure 4 is a perspective view showing how, after switching of the adhesive composition, the patient is easily able to remove adhesive dressing 100 from the forearm 14 without the need for excessive force.

## Examples

### Examples 1-5 - cross-linking component (B)

The reaction was carried out at room temperature under stirring. The components shown below in Table 1 except for the catalyst were added into a reagent bottle and mixed into a homogeneous solution, after which the catalyst was added. The mixture was left overnight for the reaction to be completed. After confirming by GPC measurements that no unreacted hydroxypropyl methacrylate or 2-ethyl-1-hexanol remained, the isocyanate functional acrylate oligomer was ready to be used.

GPC was carried out by diluting samples with tetrahydrofuran in a ratio of 1:100 and injected in an amount of 20 $\mu$l into the injection valve of a Waters HPLC 1515 pump using a flow rate of 1 ml/min of tetrahydrofuran. The instrument was equipped with a Styragel HR1 column connected to a Waters 2414 refractive index detector.

The components shown in Table 1 are as follows:

A Polyisocyanate component (i)
B Solvent.
C Catalyst
D Stabiliser
E Compound (ii)
F Compound (iii)

**Table 1**

| Reagent | | Ex. 1 | Ex 2 | Ex. 3 | Ex. 4 | Ex. 5 |
|---|---|---|---|---|---|---|
| A | Baymedix AP536 (i) (Y = 3.2) | 115.8 g | 109.2 g | 54.7 g | 54.7 g | 54.8 g |
| B | Ethyl acetate | 24.43 g | 32 g | 16.0 g | 16.0 g | 16.0 g |
| C | Zirconium (IV) pentanedionate | 0.01 g | 0.01 g | 0.01 g | 0.01 g | 0.01 g |
| D | Irganox 1010 | 0.7 g | 0.50 g | 0.25 g | 0.25 g | 0.25 g |
| E | Hydroxypropyl methacrylate (ii) | 59.1 g | 16.2 g | 4.00 g | 3.02 g | 2.03 g |
| F | 2-Ethyl-1-hexanol (iii) | 0 g | 36.2 g | 21.8 g | 22.8 g | 23.6 g |
| Degree of subst. of polyisocyanate (i) by compound (ii) | | 0.650 | 0.189 | 0.093 | 0.07 | 0.047 |
| Total degree of subst. of polyisocyanate component (i) by (ii) and (iii) | | 0.650 | 0.655 | 0.654 | 0.657 | 0.653 |

### Example 6 - Preparation of polymer component (A)

Under protection from ultraviolet sources all components in Table 2 except for Baymedix AR602 were loaded into a sealable glass jar and mixed until all solid materials had been dissolved using a magnetic stirrer. Baymedix AR602 was then added and the mixture was stirred until it become homogenous.

The components shown in Table 2 are as follows:

H Polyol
I Photoinitiator
B Solvent
C Catalyst
D Stabilizer for preventing premature switch during storage
J Surfactant

**Table 2**

|  | Reagent | Example 6 |
|---|---|---|
| H | BaymedixAR602 (X = 4) | 1797 g |
| I | Irgacure 369 | 12 g |
| B | Ethyl acetate | 190 g |
| C | Borchi Kat 22 | 2.5 g |
| D | Irganox 1010 | 2 g |
| J | BYK377 | 2.45 |

**Examples 7 to 11 - Switchable adhesive compositions**

Examples 7 to 11 are examples of switchable adhesive compositions in accordance with the present invention formulated to include the premix composition comprising cross-linking component (B) from Examples 1 to 5 and the premix composition comprising polymer component (A) from Example 6 in the amounts shown in Table 3.

Both components in Examples 7 to 11 were loaded into a sealable glass jar and mixed to a homogenous solution using a magnetic stirrer over a period of approximately 10 minutes under protection from ultraviolet sources. The resulting adhesive solution was then spread onto a flexible medical polyurethane film having a removable carrier film (medical film 48938) using a spreader having a gauge of 150 $\mu$m. The adhesive coating was then cured in a ventilated fan assisted oven at 130°C for 10 minutes. After this step, the thickness of the adhesive coating was about 60-80 $\mu$m.

**Table 3**

|  | Ex. 7** | Ex. 8 | Ex. 9 | Ex. 10 | Ex. 11 |
|---|---|---|---|---|---|
| **Cross-linking component (B)** | Ex. 1 9.81 g | Ex. 2 11.6 g | Ex. 3 13.3 g | Ex. 4 12.4 g | Ex. 5 12.5 g |
| **Polymer component (A)** | Ex. 6 28.9 g | Ex. 6 33.1 g | Ex. 6 33.1 g | Ex. 6 33.2 g | Ex. 6 33.2 g |
| **NCO/OH\*** | 0.643 | 0.635 | 0.742 | 0.680 | 0.696 |

\* NCO/OH represents the molar ratio of unsubstituted isocyanate functions in the cross-linking component (B) to nucleophilic hydroxyl groups in the polymer component (A)
\*\* Example not according to the invention.

**Peel force measurements**

Peel force before and after switching was determined for each of the adhesives of Examples 7 to 11. In preparation for peel force measurements, a very easy release liner was transferred to the exposed side of the adhesive. The removable carrier film was then removed from the medical film and replaced by a high adhesion PET tape. The PET film is fixed to the medical film in order to cancel out the elasticity effect of the medical film on the measured peel force.

Peel strengths were determined after a dwell time of 20 minutes using an Instron 5943 testing rig, equipped with a 100 N load cell, according to FINAT test method FTM1, with the exception that high density polyethylene (HDPE) panels were used as the substrate surface and that a peeling rate of 100 mm/min, crosshead speed 200 mm/s, was used in order to collect all of the necessary data within the time frame of one peel force measurement.

Adhesive switching was achieved by exposing the adhesive (adhered to the HDPE plate) to light through the PET tape and medical film backing with a light intensity of approximately 5 mW/cm2 from a XeLED-Ni3UV-R4-365-E27-SS lamp having a narrow spectrum around 365 nm. Switching times for the different coatings were measured as the time between the starting time of irradiation and the time when the substantially instantaneous loss of tack occurred, during a continuous peel strength test of about 1.5 minutes (i.e., the adhesive was peeled for a period of time whilst being irradiated). The peel force measurement was continued under irradiation until the peel force reached a plateau value, which typically occurred 5-10 seconds after the switch time. Peel strengths and switch times were measured in quadruple and the average values of switch time and peel strength (before and after switch) are reported in Table 4.

Results of the peel force measurements are shown in Table 4 alongside the meq curable groups (i.e. the olefin moiety of the methacrylate esters). The meq of curable groups is easily calculated from the mmol of hydroxypropyl methacrylate added to the adhesive and the total dry weight of the adhesive (i.e. excluding the solvent).

**Table 4**

| Example | Peel force before switch (N/25mm) | Peel force after switch (N/25mm) | Switch time (s) | Methacrylate meq/g (mmol HPMA/g dry adhesive) |
|---|---|---|---|---|
| 7 | 1.56 | 0.025 | 1.8 | 0.578 |
| 8 | 1.61 | 0.020 | 1.8 | 0.167 |
| 9 | 1.12 | 0.045 | 2.2 | 0.093 |
| 10 | 2.64 | 0.203 | 2 | 0.067 |
| 11 | 1.86 | 0.297 | 2.1 | 0.045 |

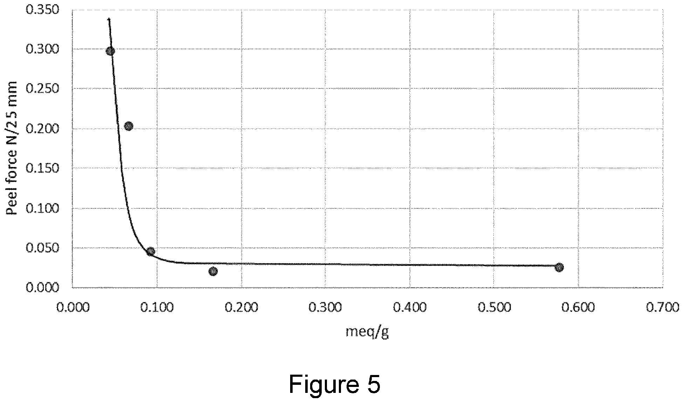

From Table 4, it can be seen that a reduction in peel force of over 80% is obtained in all cases. As expected, the peel force after switching is highest for the adhesive of Example 11, which has the lowest content of the curable methacrylate moieties. However, the reduction in peel force is nonetheless significant even when the content of curable groups is relatively low. Once the methacrylate content of the adhesive exceeds ca. 0.05 meq/g the reduction in peel force easily exceeds 90%. A comparison of Examples 7 and 8 shows that the switched peel force is essentially once the content of curable groups is above ca. 0.1 meq/g. This demonstrates that excessive amounts of curable groups are not essential to good switching performance. This provides the possibility of replacing some of the curable groups with non-curable groups from the compound (iii), without impairing (or even improving) the performance of the adhesive. In examples 7 and 8 the reduction in peel force is close to 99%, demonstrating that the switched adhesive may be removed from a substrate with essentially no damage to the substrate (and no pain in the case of removal of the switched adhesive from the skin). The results of the peel force measurements are shown graphically in Figure 5.

**Examples 12 to 17** - **Cross-linking components having different degrees of substitution of the isocyanate**

Premix compositions comprsing cross-linking components (B) having different degrees of substitution of the polyisocyanate component (i) were prepared according to the procedure set out in example 1, using the amounts of the different components set out in Table 5.

**Table 5**

| | | Reagent | Ex. 12 | Ex. 13 | Ex. 14 | Ex. 15 | Ex. 16 | Ex. 17 |
|---|---|---|---|---|---|---|---|---|
| | A | Baymedix AP536 (i) (Y = 3.2) | 71.0 g | 51.4 g | 40.0 g | 51.2 g | 40.0 g | 71.0 g |
| | B | Ethyl acetate | 12.4 g | 10.7 g | 9.0 g | 11.5 g | 9.0 g | 17.0 g |
| | C | Dibutylin dilaurate | 0.01 g | 0.01 | 0.01 g | 0.01 g | 0.01 g | 0.01 g |
| | D | Irganox 1010 | 0.33 g | 0.33 g | 0.25 g | 0.36 g | 0.24 g | 0.45 g |
| | E | Hydroxypropyl methacrylate (ii) | 14.0 g | 20.1 g | 19.0 g | 26.3 g | 22.0 g | 42.0 g |
| **Degree of substitution (%)** | | | 25.1 | 49.8 | 60.5 g | 65.4 g | 70.0 g | 75.3 g |
| Degree of subst. of polyisocyanate (i) by compound (ii) | | | 0.251 | 0.498 | 0.605 | 0.654 | 0.700 | 0.753 |
| Total degree of subst. of polyisocyanate component (i) by (ii) and (iii) | | | 0.251 | 0.498 | 0.605 | 0.654 | 0.700 | 0.753 |

**Examples 18 to 31** - **Switchable adhesive compositions**

Examples 18 to 31 are examples of switchable adhesive compositions in accordance with the present invention formulated to include the premix compositions comprising cross-linking components (B) from Examples 12 to 17 and the premix compositions comprising polymer component (A) from Example 6 in the amounts shown in Table 6. The adhesives were formulated according to the method of Example 7, except that the adhesives were spread onto film using a gauge of 200 $\mu$m, resulting in adhesive coatings with a coat weight of 90-100 g/m$^2$. Peel force was measured as described above in quadruple samples per adhesive and is reported in Table 6. The measured values represent adhesive failure. No cohesive failure was observed in these tests. In other words, the maximum measured peel force represents the force necessary to separate the adhesive from the substrate and not internal breakdown of the adhesive structure (which would leave a residue on the surface).

It will be appreciated that as the degree of substitution of the polyisocyanate component increases, it is necessary to increase the relative amount of the cross-linking component (B) to ensure sufficient unreacted isocyanate groups are available for reaction with the polymer component (A).

**Table 6**

| Ex. | (B) from | Degree of substitution | Amount (A) /g | Amount (B) /g | Ratio* | Peel force before switch (N/25mm) | NCO/ OH |
|---|---|---|---|---|---|---|---|
| 18** | Ex.12 | 25% | 33.00 | 2.19 | 0.31 | 0.89 | 0.316 |
| 19** | Ex. 13 | 50% | 29.02 | 4.40 | 0.44 | 1.03 | 0.429 |
| 20** | Ex. 13 | 50% | 29.02 | 4.21 | 0.42 | 1.21 | 0.408 |
| 21 ** | Ex. 14 | 60% | 33.04 | 8.15 | 0.53 | 2.74 | 0.526 |
| 22** | Ex. 14 | 60% | 33.03 | 8.41 | 0.55 | 2.04 | 0.545 |
| 23** | Ex. 14 | 60% | 33.05 | 8.71 | 0.57 | 1.71 | 0.565 |
| 24** | Ex. 15 | 65% | 29.00 | 9.00 | 0.57 | 4.27 | 0.570 |
| 25** | Ex. 15 | 65% | 28.99 | 9.57 | 0.61 | 2.25 | 0.609 |
| 26** | Ex. 15 | 65% | 29.00 | 9.41 | 0.60 | 3.18 | 0.598 |
| 27** | Ex. 16 | 70% | 29.02 | 13.00 | 0.71 | 4.55 | 0.709 |
| 28** | Ex. 16 | 70% | 29.02 | 13.54 | 0.74 | 3.47 | 0.740 |
| 29** | Ex. 16 | 70% | 29.04 | 14.03 | 0.77 | 2.57 | 0.768 |
| 30** | Ex. 17 | 75% | 25.01 | 18.99 | 0.94 | 6.88 | 0.975 |
| 31 ** | Ex. 17 | 75% | 25.00 | 19.21 | 0.95 | 7.07 | 0.987 |
| * Ratio of unsubstituted isocyanate functions in the cross-linking component (B) to nucleophilic groups in the polymer component (A) <br> ** Not according to the invention. | | | | | | | |

As shown in Table 6, the degree to which the isocyanate functions of the cross-linking component (B) are substituted with either curable groups (such as acrylate/methacrylate) or non-curable groups affects the adhesive properties of the adhesive composition. While it might be expected that the cross-linking component (B) could be made simply with a sufficient amounts of unsaturated moieties and excluding the non-curable moieties (from compound (iii)), it is found that the adhesiveness of the formed composition is lower when the cross-linking component (B) has only a low degree of substitution. The distribution and density of cross links in the formed polymer matrix, and thus the properties of the adhesive, can be tuned for a polyol-isocyanate oligomer pair, or mix thereof, by substituting part of the isocyanate component (i) with a non-curable compound (iii). Accordingly, the minimum degree of substitution of the polyisocyanate component (i) is at least 0.2, and preferably at least 0.3, more preferably at least 0.4, and most preferably at least 0.45 or at least 0.5.

On the other hand, while a strong adhesive may be formed using cross-linking components (B) with very high levels of isocyanate substitution, the amount of cross-linking component (B) required to form sufficient cross-links with the polymer component (A) is very high, which is not cost-effective. For example, the maximum degree of substitution of the polyisocyanate component (i) is no more than the lesser of 0.3Y and 0.8, for example no more than the lesser of 0.28Y and 0.75.

**Example 32** - **Pumping of premix composition comprising cross-linking component (B)**

The ratio between the polymer component (A) and cross-linking component (B) will decide the tackiness of the adhesive, and at the same time a small variation of the ratio may have a large impact on the adhesion performance. Therefore, one of the best ways to sustain a stable ratio during coating of the adhesive is to use a cogwheel pump (gear metering pump). In the examples below a 2K GMM e2 mixer from Scanrex Industriservice AB equipped with a GM301D-30R-110Z 3.0 cc and a GM601D-30R-110Z 6.0cc pumps from Oerlikon Barmag for the cross-linking and polymer component, respectively.

In a production trial, 20 kg of the premix composition comprising cross-linking component (B) from Example 1 (ca. 65% substitution of isocyanate by curable groups) and 40 kg of the premix composition comprising polymer component (A) from Example 6 were prepared and charged into their respective vessels. The mixer was set at a total flow rates displayed below in Table 7. The mixing process was then started and proceeded under intermittent conditions until the cross-linking component (B) 3.0 cc pump stopped due to too high torque. When dissembling the cross-linking component pump head, a white rubber-like material was noticed on the cogwheel as well as on the pump house surrounding it. Since it is well known that high shear forces can initiate polymerization it is believed that this was the reason to the clogging of the pump.

In a second trial, 5 kg of the premix composition comprising the cross-linking component made according to Example 2 (ca. 19% substitution of isocyanate by curable groups) was charged into its corresponding vessel where after arranging a recirculation setup the cross-linking component pump (with the polymer component pump disconnected) was started at a flow rate of 2.5 ml/s. After pumping 970 litres around in the system without any pump failure, no visible matters could be detected in the pump head after dissembling it which clearly demonstrates that polymerization can be avoided in the pump head by keeping the amounts of curable moieties below a certain concentration, while maintaining the total degree of substitution of the polyisocyanate component (i) by way of the compound (iii).

**Table 7**

| Cross-linking component (B) | Flow rate ml/s | Pumped volume L | Running condition | Pump head condition after stop |
|---|---|---|---|---|
| Example 1 | 2.4 | 5 | Intermittent | Clogged |
| Example 1 | 1.3 | 9 | Intermittent | Clogged |
| Example 1 | 1.3 | 13 | Intermittent | Clogged |
| Example 2 | 2.5 | 970 | Continuous | Ok |

**Example 33 - Adhesives including a photoinitiator as compound (iii)**

A premix composition comprising cross-linking component (B) comprising a photoinitiator as compound (iii) was prepared by the method of example 1 using the components set out in Table 8.

**Table 8**

| | Reagent | Amount (g) |
|---|---|---|
| A | Baymedix AP536 (i) (Y = 3.2) | 103.11 |
| B | Ethyl acetate | 29.3 |
| C | Zirconium (IV) pentanedionate | 0.04 |
| D | Irganox 1010 | 0.39 |
| E | Hydroxypropyl methacrylate (ii) | 15.01 |
| F | 2-Ethyl hexanol (iii) | 20.89 |
| I | Irgacure 127* | 20.00 |
| Degree of subst. of polyisocyanate (i) by compound (ii) | | 0.185 |
| Total degree of subst. of polyisocyanate component (i) by (ii) and (iii) | | 0.679 |
| * hydroxyl-substituted photoinitiator | | |

A premix composition comprising polymer component (A) was prepared by the method of example 6 using the components set out in Table 9.

**Table 9**

|   | Reagent | Amount (g) |
|---|---------|-----------|
| H | Baymedix AR602 (X = 4) | 719.1 |
| B | Ethyl acetate | 76.01 |
| C | Borchi Kat 22 | 1.04 |
| D | Irganox 1010 | 1.15 |
| J | BYK 377 | 1.04 |

An adhesive composition was prepared by combining 33.9 g of the polymer component (A) and 9.94 g of the cross-linking component (B) according to the method of Example 7. The NCO/OH ratio of this adhesive was 0.465. The adhesive gave an initial peel force of 1.45 N/25mm, a peel force after switch of 0.10 N/25mm and a switch time of 9.6 s on HDPE. A particular advantage of the adhesive of example 33 is that the potentially hazardous photoinitiator becomes chemically bonded to the adhesive polymer matrix such that it is unable to be absorbed by the skin.

**Example 34 -Adhesives including a photoinitiator as compound (iii)**

A premix composition comprising cross-linking component (B) comprising a photoinitiator as compound (iii) was prepared by the method of example 1 using the components set out in Table 10.

**Table 10**

|   | Reagent | Amount (g) |
|---|---------|-----------|
| A | Baymedix AP536 (i) (Y = 3.2) | 46.6 |
| B | Ethyl acetate | 13.3 |
| C | Zirconium (IV) pentanedionate | 0.01 |
| D | Irganox 1010 | 0.19 |
| E | Hydroxypropyl methacrylate (ii) | 8 |
| F | 2-Ethyl hexanol (iii) | 5.6 |
| I | Omnirad 2959* | 10.6 |
| Degree of subst. of polyisocyanate (i) by compound (ii) | | 0.219 |
| Total degree of subst. of polyisocyanate component (i) by (ii) and (iii) | | 0.574 |
| * hydroxyl-substituted photoinitiator | | |

A premix composition comprising polymer component (A) was prepared by the method of example 6 using the components set out in Table 11.

**Table 11**

|   | Reagent | Amount (g) |
|---|---------|-----------|
| H | Baymedix AR602 (X = 4) | 900.8 |
| B | Ethyl acetate | 66.7 |
| C | Borchi Kat 22 | 1.1 |

An adhesive composition was prepared by combining 20.05 g of the premix comprising polymer component (A) and 7.82 g of the premix composition comprising cross-linking component (B) according to the method of Example 7. The NCO/OH ratio of this adhesive was 0.841. The adhesive gave an initial peel force of 2.9 N/25mm, a peel force after switch of 0.08 N/25mm and a switch time of 5.5 s on HDPE. A particular advantage of the adhesive of example 34 is again that the potentially hazardous photoinitiator becomes chemically bonded to the adhesive polymer matrix such that it is unable to be absorbed by the skin.

**Examples 35 to 38** - **Cytotoxicity of the adhesive**

A premix comprising cross-linking component (B) was prepared by the method of Example 1 using the components set out in Table 12.

**Table 12 (Example 35)**

|   | Reagent | Amount (g) |
|---|---------|-----------|
| A | Baymedix AP536 (i) (Y = 3.2) | 51.3 |
| B | Ethyl acetate | 10.8 |
| C | Zirconium (IV) pentanedionate | 0.002 |
| D | Irganox 1010 | 0.3 |
| E | Hydroxypropyl methacrylate (ii) | 26.2 |
| F | 2-Ethyl-1-hexanol (iii) | 0 |
| Degree of subst. of polyisocyanate (i) by compound (ii) | | 0.650 |
| Total degree of subst. of polyisocyanate component (i) by (ii) and (iii) | | 0.650 |

A premix comprising polymer component (A) was prepared by the method of Example 6 using the components set out in Table 13.

**Table 13 (Example 36)**

|   | Reagent | Amount (g) |
|---|---------|-----------|
| H | Baymedix AR602 (X = 4) | 88.0 |
| I | Irgacure 369 | 0 |
| B | Ethyl acetate | 9.0 |
| C | Borchi Kat 22 | 0.06 |
| D | Irganox 1010 | 0 |
| J | BYK 377 | 0.13 |

Adhesives were formulated according to the method of Example 7 using a premix composition comprising polymer components (A) and a premix composition comprising cross-linking components (B) in the proportions set out in Table 14.

**Table 14**

|   | Example 37** | Example 38 |
|---|--------------|-----------|
| **Premix composition comprising a cross-linking component (B)** | Ex. 35 11.85 g | Ex. 2 12.15 g |
| **Premix composition comprising a polymer component (A)** | Ex. 36 28.9 g | Ex. 6 33.0 g |
| **NCO/OH** | 0.777 | 0.669 |
| ** Not according to the invention. | | |

The cytotoxicity of the Examples 37 and 38 (measured according to cytotoxicity tests complying with the methods described in the guidelines of ISO 10993-5, Biological Evaluation of Medical Devices) is set out in Table 15. To determine whether less concentrated examples would show more favourable cytotoxicity, the adhesive composition extracts were diluted. In Table 15, dilution ratio indicates to what extent the extract was diluted before performing the cytotoxicity test. The ratio extract:culture media is tabulated. It should be noted that it is only if the undiluted, 1:0, sample shows a viability above 70% that decides if the sample has passed the test or not.

**Table 15**

| Example | Dilution ratio | | | |
|---|---|---|---|---|
| | Undiluted | 1:2 | 1:4 | 1:8 |
| **38** | 75 | 81 | 90 | 94 |
| **37\*\*** | 39 | 73 | 83 | 88 |
| \*\* Not according to the invention. | | | | |

As can be seen in Table 15, the sample from example 38 passed the cytotoxicity test whilst the sample from example 37 failed, despite its lack of photoinitiator and approximate halved catalyst concentration, both of which were expected to exert a certain toxicity to living cells. This demonstrates that a reduction in the concentration of curable moieties in the adhesive has a beneficial effect on the cytotoxicity of the adhesive, without impairing the switching properties of the adhesive.

In circumstances in which the medical skin covering will be in contact with a patient's skin for a prolonged period, or in which the medical skin covering will be replaced with a new medical skin covering several times over a prolonged period, it is particularly important for the switchable adhesive compositions that are used in the medical skin coverings to be of low cytotoxicity.

**Example 39**

A premix composition comprising cross-linking component (B) was prepared by the method of Example 1 using the components set out in Table 16.

**Table 16**

| | Reagent | Amount (g) |
|---|---|---|
| **A** | Baymedix AP501 (i) (Y =2) | 63.29 |
| **B** | Ethyl acetate | 10.00 |
| **C** | Zirconium (IV) pentanedionate | 0.02 |
| **D** | Irganox 1010 | 0.1 |
| **E** | Hydroxypropyl methacrylate (ii) | 7.07 |
| Degree of subst. of polyisocyanate (i) by compound (ii) | | 0.252 |
| Total degree of subst. of polyisocyanate component (i) by (ii) and (iii) | | 0.252 |

**Example 40\*\***

An adhesive was formulated according to the method of Example 7 using the premix composition comprising polymer component (A) of Example 6 and premix composition comprising cross-linking component (B) of Example 39 in the proportions set out in Table 17.

**Table 17**

| Premix composition comprising cross-linking component (B) | Example 39 6.38 g |
|---|---|
| Premix composition comprising polymer component (A) | Example 6 33.17 g |
| Premix composition comprising cross-linking component (B) | Example 39 6.38 g |
| NCO/OH | 0.592 |

The adhesive gave an initial peel force of 3.38 N/25mm, a peel force after switch of 0.03 N/25mm and a switch time of 1.8 s on HDPE. An advantage of adhesives produced in combination with cross-linking components (B) similar to those in example 39 is that considerably less amount of the isocyanate component is required. This is more cost effective as the isocyanate component is expensive in relation to the polymer component (A).

** Not according to the invention.

**Materials**

The following materials were used in the examples described above.

| Material [function] | Description | Supplier |
|---|---|---|
| Baymedix AR602 [Polymer component A] | Tetra-functional hydroxy-terminated polyether polyol; equivalent weight per hydroxyl group of ca. 1600 Da. X = 4 | Covestro AG |
| Baymedix AP501 [Polyisocyanate component (i)] | Aliphatic NCO terminated prepolymer based on hexamethylene diisocyanate NCO content 12.8 w/w% Y = 2 | Covestro AG |
| Baymedix AP536 [Polyisocyanate component (i)] | Trimerized hexamethylene diisocyanate NCO content 23 w/w% Y = 3.2 | Covestro AG |
| Borchi Kat 22 | Polyurethane catalyst | Borchers |
| BYK 377 | Surfactant | BYK-Chemie GMBH |
| Songnox1010 | Pentaerythritol Tetrakis(3-(3,5-di-tert-butyl-4-hydroxyphenyl) propionate (antioxidant) | IGM Resins |
| Omnirad 127 | 2-hydroxy-1-{4-[4-(2-hydroxy-2-methyl-propionyl)-benzyl]-phenyl}-2-methylpropan-1-one (photoinitiator) | IGM Resins |
| Omnirad 2959 | 1-[4-(2-Hydroxyethoxyl)-phenyl]-2-hydroxy-2-methylpropanone (photoinitiator) | IGM Resins |
| Omnirad 369 | 2-Benzyl-2-(dimethylamino)-4'-morpholinobutyrophenone (photoinitiator) | BASF AG |
| Code 48938 | Medical film | Shanghai ISO Medical Products Co. Ltd. |
| HDPE panels | Panels for peel test measurements | ChemInstrument Inc. |
| Release liner | Transparent PET Release film, 50 $\mu$m, silicone 1720, quality 1876 | Huhtamaki Oyj |

**Claims**

1.  An adhesive polyurethane composition comprising the reaction product of:

    (A) a polymer component having a weight average molecular weight in the range of 500 to 100,000 Dalton, and containing an average per molecule of X nucleophilic functional groups containing an active hydrogen atom, wherein X represents a number having a value of at least 2; and
    (B) a cross-linking component obtained by reacting:

       (i) a polyisocyanate component having an average of at least Y isocyanate functions per molecule, wherein Y represents a number in the range from 1.8 to 6;
       (ii) at least one compound comprising a functional group that is curable by free-radical polymerisation and further comprising a nucleophilic functional group containing an active hydrogen atom; and
(iii) at least one compound comprising a nucleophilic functional group containing an active hydrogen atom and which does not include a functional group that is curable by free-radical polymerisation;

wherein the total degree of substitution of the polyisocyanate component (i) by the compounds (ii) and (iii) is at least 0.2 and no more than the lesser of 0.3Y and 0.8.

2. An adhesive polyurethane composition according to claim 1, wherein the total degree of substitution of the polyisocyanate component (i) by the compounds (ii) and (iii) is at least 0.3, preferably at least 0.4.

3. An adhesive polyurethane composition according to any preceding claim, wherein the cross-linking component (i) is obtained by reacting the polyisocyanate component (i) with both the compound (ii) and the compound (iii).

4. An adhesive polyurethane composition according to claim 3, wherein the degree of substitution of the polyisocyanate component (i) by the compound (ii) is no more than 0.6, preferably no more than 0.55, preferably no more than 0.5, preferably no more than 0.4, preferably no more than 0.3, preferably no more than 0.25, preferably no more than 0.2, preferably no more than 0.18; and/or wherein the degree of substitution of the polyisocyanate component (i) by the compound (ii) is at least 0.05, preferably at least 0.06, preferably at least 0.08, preferably at least 0.1, preferably at least 0.12.

5. An adhesive polyurethane composition according to any preceding claim, wherein Y represents a number in the range from 2 to 4, preferably in the range from 2 to 3.6, preferably in the range from 2.1 to 3.5, preferably in the range from 2.2 to 3.4; and/or wherein X represents a number of at least 2.2, preferably at least 2.4, preferably at least 2.6, preferably at least 2.8, preferably at least 3.

6. An adhesive polyurethane composition according to any preceding claim, wherein the polyisocyanate component (i) comprises one or more polyisocyanate compounds selected from the group of polyisocyanates containing from 2 to 10 isocyanate functions per molecule.

7. An adhesive polyurethane composition according to claim 6, wherein the polyisocyanate component (i) comprises a mixture of a diisocyanate and at least one polyisocyanate having an average of at least 3 isocyanate functions per molecule.

8. An adhesive polyurethane composition according to claim 6 or claim 7, wherein the polyisocyanate component (i) comprises a trimerized diisocyanate of the formula $D(R-NCO)_3$, wherein D represents a ring structure selected from isocyanurate and iminooxadiazindione and mixtures thereof, and each R independently represents a straight chain, branched or cyclic alkylene group having from 2 to 15 carbon atoms or an aryl group having from 6 to 20 carbon atoms, preferably wherein the at least one polyisocyanate is trimerized hexamethylene diisocyanate.

9. An adhesive polyurethane composition according to any preceding claim, wherein the at least one compound (ii) comprises an olefin moiety as the functional group that is curable by free-radical polymerisation; preferably wherein the at least one compound (ii) is selected from hydroxy-substituted acrylate esters, hydroxy-substituted methacrylate esters, and mixtures thereof; preferably wherein the at least one compound (ii) is selected from hydroxy-$(C_2-C_{20})$alkyl-substituted methacrylate esters, polyalkoxylated monomethacrylate containing 2-10 ether functions, and mixtures thereof; more preferably wherein the compound (ii) is selected from 2-hydroxypropyl methacrylate, hydroxyethyl methacrylate and mixtures thereof.

10. An adhesive polyurethane composition according to any preceding claim, wherein the at least one compound (iii) comprises or consists of one or more $C_1-C_{30}$ aliphatic alcohols, preferably linear, branched or cyclic $C_1-C_{18}$ aliphatic alcohols, preferably linear, branched or cyclic $C_1-C_{12}$ aliphatic alcohols, preferably branched $C_3-C_{12}$ aliphatic alcohols, or branched $C_6-C_{18}$ aliphatic alcohols.

11. An adhesive polyurethane composition according to any preceding claim, wherein the at least one compound (iii) comprises or consists of one or more hydroxy-substituted photoinitiators.

12. An adhesive polyurethane composition according to any preceding claim, wherein the polymer component (A) is selected from hydroxy-terminated polyethers, hydroxy-terminated polyesters, amine-terminated polyethers, and amine-terminated polyesters, preferably wherein the polymer component is a hydroxy-terminated polyether; preferably wherein the hydroxy-terminated polyether is selected from alkoxylated derivatives of compounds comprising from two to five hydroxy groups, or mixtures thereof;

preferably wherein the hydroxy-terminated polyether comprises alkoxylated derivatives of ethylene glycol, propylene glycol, butane-1,4-diol, glycerol, trimethylolpropane, erythritol, pentaerythritol, pentane-1,2,4,5-tetrol, dextrose and sorbitol, preferably wherein the alkoxylated derivatives are ethoxylated derivates, propoxylated derivatives, or ethoxylated-co-propoxylated derivatives.

13. An adhesive polyurethane composition according to any preceding claim, wherein the polymer component has a weight average molecular weight in the range of 1,000 to 50,000 Dalton, preferably in the range of 1,000 to 20,000 Dalton, preferably in the range of 1,500 to 10,000 Dalton; and/or wherein the polymer component has an equivalent weight per nucleophilic functional group of from 200 to 5,000, preferably from 500 to 2,000, preferably from 1,000 to 2,000.

14. A method of preparing an adhesive polyurethane composition comprising:

(a) a first step of reacting:

(i) a polyisocyanate component having an average of at least Y isocyanate functions per molecule, wherein Y represents a number in the range from 1.8 to 6; with
(ii) at least one compound comprising a functional group that is curable by free-radical polymerisation and further comprising a nucleophilic functional group containing an active hydrogen atom; and
(iii) at least one compound comprising a nucleophilic functional group containing an active hydrogen atom and which does not include a functional group that is curable by free-radical polymerisation;

wherein the total degree of substitution of the polyisocyanate by the compounds (ii) and (iii) is at least 0.2 and no more than the lesser of 0.3Y and 0.8, to form a cross-linking component (B);
(b) a second step of combining the product of step (i) with a polymer component (A) having a weight average molecular weight in the range of 500 to 100,000 Dalton, and containing an average per molecule of X nucleophilic functional groups containing an active hydrogen atom, wherein X represents a number greater than 2.

15. An adhesive medical product comprising a switchable adhesive polyurethane composition as defined in any of claims 1 to 13 disposed between a first carrier film and a release liner; preferably wherein the first carrier film is UV translucent, optionally wherein a removable UV occlusive layer is laminated to the first carrier film on the surface opposite the adhesive composition.

**Patentansprüche**

1. Polyurethan-Klebstoffzusammensetzung, die das Reaktionsprodukt umfasst von:

a) einer Polymerkomponente, die ein gewichtsmittleres Molekulargewicht im Bereich von 500 bis 100.000 Dalton aufweist und einen Durchschnitt pro Molekül von X nukleophilen funktionellen Gruppen enthält, die ein aktives Wasserstoffatom enthalten, wobei X eine Zahl darstellt, die einen Wert von mindestens 2 aufweist; und
b) einer Vernetzungskomponente, die erhalten wird durch Reagieren:

(i) einer Polyisocyanat-Komponente, die einen Durchschnitt von mindestens Y Isocyanat-Funktionen pro Molekül aufweist, wobei Y eine Zahl im Bereich von 1,8 bis 6 darstellt;
(ii) mindestens einer Verbindung, die eine funktionelle Gruppe umfasst, die durch radikalische Polymerisation härtbar ist, und weiter eine nukleophile funktionelle Gruppe umfasst, die ein aktives Wasserstoffatom enthält; und
(iii) mindestens einer Verbindung, die eine nukleophile funktionelle Gruppe umfasst, die ein aktives Wasserstoffatom enthält, und die keine funktionelle Gruppe beinhaltet, die durch radikalische Polymerisation härtbar ist;

wobei der Gesamtsubstitutionsgrad der Polyisocyanat-Komponente (i) durch die Verbindungen (ii) und (iii) mindestens 0,2 und nicht mehr als das kleinere von 0,3Y und 0,8 beträgt.

2. Polyurethan-Klebstoffzusammensetzung nach Anspruch 1, wobei der Gesamtsubstitutionsgrad der Polyisocyanat-Komponente (i) durch die Verbindungen (ii) und (iii) mindestens 0,3, vorzugsweise mindestens 0,4 beträgt.

3. Polyurethan-Klebstoffzusammensetzung nach einem vorstehenden Anspruch, wobei die Vernetzungskomponente (i) durch Reagieren der Polyisocyanat-Komponente (i) mit beiden, der Verbindung (ii) und der Verbindung (iii) erhalten wird.

4. Polyurethan-Klebstoffzusammensetzung nach Anspruch 3, wobei der Substitutionsgrad der Polyisocyanat-Komponente (i) durch die Verbindung (ii) nicht mehr als 0,6, vorzugsweise nicht mehr als 0,55, vorzugsweise nicht mehr als 0,5, vorzugsweise nicht mehr als 0,4, vorzugsweise nicht mehr als 0,3, vorzugsweise nicht mehr als 0,25, vorzugsweise nicht mehr als 0,2, vorzugsweise nicht mehr als 0,18 beträgt; und/oder wobei der Substitutionsgrad der Polyisocyanat-Komponente (i) durch die Verbindung (ii) mindestens 0,05, vorzugsweise mindestens 0,06, vorzugsweise mindestens 0,08, vorzugsweise mindestens 0,1, vorzugsweise mindestens 0,12 beträgt.

5. Polyurethan-Klebstoffzusammensetzung nach einem vorstehenden Anspruch, wobei Y eine Zahl im Bereich von 2 bis 4, vorzugsweise im Bereich von 2 bis 3,6, vorzugsweise im Bereich von 2,1 bis 3,5, vorzugsweise im Bereich von 2,2 bis 3,4 darstellt; und/oder wobei X eine Zahl von mindestens 2,2, vorzugsweise mindestens 2,4, vorzugsweise mindestens 2,6, vorzugsweise mindestens 2,8, vorzugsweise mindestens 3 darstellt.

6. Polyurethan-Klebstoffzusammensetzung nach einem vorstehenden Anspruch, wobei die Polyisocyanat-Komponente (i) eine oder mehrere Polyisocyanat-Verbindungen umfasst, ausgewählt aus der Gruppe von Polyisocyanaten, die 2 bis 10 Isocyanat-Funktionen pro Molekül enthalten.

7. Polyurethan-Klebstoffzusammensetzung nach Anspruch 6, wobei die Polyisocyanat-Komponente (i) ein Gemisch aus einem Diisocyanat und mindestens einem Polyisocyanat umfasst, das einen Durchschnitt von mindestens 3 Isocyanat-Funktionen pro Molekül aufweist.

8. Polyurethan-Klebstoffzusammensetzung nach Anspruch 6 oder Anspruch 7, wobei die Polyisocyanat-Komponente (i) ein trimerisiertes Diisocyanat der Formel $D(R-NCO)_3$ umfasst, wobei D eine Ringstruktur, ausgewählt aus Isocyanurat und Iminooxadiazindion und Gemischen davon, darstellt, und jedes R unabhängig eine geradkettige, verzweigte oder zyklische Alkylengruppe, die 2 bis 15 Kohlenstoffatome aufweist, oder eine Arylgruppe, die 6 bis 20 Kohlenstoffatome aufweist, darstellt, wobei das mindestens eine Polyisocyanat vorzugsweise trimerisiertes Hexamethylendiisocyanat ist.

9. Polyurethan-Klebstoffzusammensetzung nach einem vorstehenden Anspruch, wobei die mindestens eine Verbindung (ii) einen Olefinrest als funktionelle Gruppe umfasst, die durch radikalische Polymerisation härtbar ist; wobei die mindestens eine Verbindung (ii) vorzugsweise aus hydroxy-substituierten Acrylatestern, hydroxy-substituierten Methacrylatestern und Gemischen davon ausgewählt ist; wobei die mindestens eine Verbindung (ii) vorzugsweise aus Hydroxy-$(C_2-C_{20})$-alkyl-substituierten Methacrylatestern, polyalkoxyliertem Monomethacrylat, das 2-10 Etherfunktionen enthält, und Gemischen davon ausgewählt ist; wobei die Verbindung (ii) bevorzugter aus 2-Hydroxypropylmethacrylat, Hydroxyethylmethacrylat und Gemischen davon ausgewählt ist.

10. Polyurethan-Klebstoffzusammensetzung nach einem vorstehenden Anspruch, wobei die mindestens eine Verbindung (iii) einen oder mehrere aliphatische $C_1$-$C_{30}$-Alkohole, vorzugsweise lineare, verzweigte oder zyklische aliphatische $C_1C_{18}$-Alkohole, vorzugsweise lineare, verzweigte oder zyklische aliphatische $C_1$-$C_{12}$-Alkohole, vorzugsweise verzweigte aliphatische $C_3$-$C_{12}$-Alkohole oder verzweigte aliphatische $C_6$-$C_{18}$-Alkohole umfasst oder aus diesen besteht.

11. Polyurethan-Klebstoffzusammensetzung nach einem vorstehenden Anspruch, wobei die mindestens eine Verbindung (iii) einen oder mehrere hydroxy-substituierte Photoinitiatoren umfasst oder aus diesen besteht.

12. Polyurethan-Klebstoffzusammensetzung nach einem vorstehenden Anspruch, wobei die Polymerkomponente (A) aus hydroxy-terminierten Polyethern, hydroxy-terminierten Polyestern, amin-terminierten Polyethern und amin-terminierten Polyestern ausgewählt ist, wobei die Polymerkomponente vorzugsweise ein hydroxyterminierter Polyether ist; wobei der hydroxy-terminierte Polyether vorzugsweise aus alkoxylierten Derivaten von Verbindungen, die zwei bis fünf Hydroxygruppen umfassen, oder Gemischen davon ausgewählt ist;
wobei der hydroxy-terminierte Polyether vorzugsweise alkoxylierte Derivate von Ethylenglykol, Propylenglykol, Butan-1,4-diol, Glycerin, Trimethylolpropan, Erythrit, Pentaerythrit, Pentan-1,2,4,5-tetrol, Dextrose und Sorbit umfasst, wobei die alkoxylierten Derivate vorzugsweise ethoxylierte Derivate, propoxylierte Derivate oder ethoxylierte-copropoxylierte Derivate sind.

13. Polyurethan-Klebstoffzusammensetzung nach einem vorstehenden Anspruch, wobei die Polymerkomponente ein gewichtsmittleres Molekulargewicht im Bereich von 1.000 bis 50.000 Dalton, vorzugsweise im Bereich von 1.000 bis 20.000 Dalton, vorzugsweise im Bereich von 1.500 bis 10.000 Dalton aufweist; und/oder wobei die Polymerkomponente ein Äquivalentgewicht pro nukleophiler funktioneller Gruppe von 200 bis 5.000, vorzugsweise von 500 bis 2.000, vorzugsweise von 1.000 bis 2.000 aufweist.

14. Verfahren zum Herstellen einer Polyurethan-Klebstoffzusammensetzung, umfassend:

a) einen ersten Schritt des Reagierens:

(i) einer Polyisocyanat-Komponente, die einen Durchschnitt von mindestens Y Isocyanat-Funktionen pro Molekül aufweist, wobei Y eine Zahl im Bereich von 1,8 bis 6 darstellt; mit
(ii) mindestens einer Verbindung, die eine funktionelle Gruppe umfasst, die durch radikalische Polymerisation härtbar ist, und weiter eine nukleophile funktionelle Gruppe umfasst, die ein aktives Wasserstoffatom enthält; und
(iii) mindestens einer Verbindung, die eine nukleophile funktionelle Gruppe umfasst, die ein aktives Wasserstoffatom enthält, und die keine funktionelle Gruppe beinhaltet, die durch radikalische Polymerisation härtbar ist;

wobei der Gesamtsubstitutionsgrad des Polyisocyanats durch die Verbindungen (ii) und (iii) mindestens 0,2 und nicht mehr als das kleinere von 0,3Y und 0,8 beträgt, um eine Vernetzungskomponente (B) zu bilden;
b) einen zweiten Schritt des Kombinierens des Produkts aus Schritt (i) mit einer Polymerkomponente (A), die ein gewichtsmittleres Molekulargewicht im Bereich von 500 bis 100.000 Dalton aufweist und einen Durchschnitt pro Molekül von X nukleophilen funktionellen Gruppen enthält, die ein aktives Wasserstoffatom enthalten, wobei X eine Zahl größer als 2 darstellt.

15. Klebendes Medizinprodukt, das eine wechselbare Polyurethan-Klebstoffzusammensetzung nach einem der Ansprüche 1 bis 13 umfasst, die zwischen einem ersten Trägerfilm und einer Trennlage angeordnet ist; wobei der erste Trägerfilm vorzugsweise UV-durchlässig ist, wobei optional eine entfernbare UV-Okklusivschicht auf der der Klebstoffzusammensetzung gegenüberliegenden Fläche auf den erste Trägerfilm auflaminiert ist.

**Revendications**

1. Composition adhésive de polyuréthane comprenant le produit de réaction de :

(A) un composant polymère présentant un poids moléculaire moyen en poids dans la plage de 500 à 100 000 Daltons, et contenant une moyenne par molécule de X groupes fonctionnels nucléophiles contenant un atome d'hydrogène actif, dans laquelle X représente un nombre présentant une valeur d'au moins 2 ; et
(A) un composant de réticulation obtenu en faisant réagir :

(i) un composant polyisocyanate présentant une moyenne d'au moins Y fonctions isocyanate par molécule, dans laquelle Y représente un nombre dans la plage de 1,8 à 6 ;
(ii) au moins un composé comprenant un groupe fonctionnel durcissable par polymérisation radicalaire et comprenant en outre un groupe fonctionnel nucléophile contenant un atome d'hydrogène actif ; et
(iii) au moins un composé comprenant un groupe fonctionnel nucléophile contenant un atome d'hydrogène actif et qui n'inclut pas de groupe fonctionnel durcissable par polymérisation radicalaire ;

dans laquelle le degré total de substitution du composant polyisocyanate (i) par les composés (ii) et (iii) est d'au moins 0,2 et inférieur ou égal au moins élevé parmi 0,3 Y et 0,8.

2. Composition adhésive de polyuréthane selon la revendication 1, dans laquelle le degré total de substitution du composant polyisocyanate (i) par les composés (ii) et (iii) est d'au moins 0,3, de préférence d'au moins 0,4.

3. Composition adhésive de polyuréthane selon une quelconque revendication précédente, dans laquelle le composant de réticulation (i) est obtenu en faisant réagir le composant polyisocyanate (i) avec à la fois le composé (ii) et le composé (iii).

4. Composition adhésive de polyuréthane selon la revendication 3, dans laquelle le degré de substitution du composant polyisocyanate (i) par le composé (ii) est inférieur ou égal à 0,6, de préférence pas supérieur à 0,55, de préférence pas supérieur à 0,5, de préférence pas supérieur à 0,4, de préférence pas supérieur à 0,3, de préférence pas supérieur à 0,25, de préférence pas supérieur à 0,2, de préférence pas supérieur à 0,18 ; et/ou dans laquelle le degré de substitution du composant polyisocyanate (i) par le composé (ii) est d'au moins 0,05, de préférence d'au moins 0,06, de préférence d'au moins 0,08, de préférence d'au moins 0,1, de préférence d'au moins 0,12.

5. Composition adhésive de polyuréthane selon une quelconque revendication précédente, dans laquelle Y représente un nombre dans la plage de 2 à 4, de préférence dans la plage de 2 à 3,6, de préférence dans la plage de 2,1 à 3,5, de préférence dans la plage de 2,2 à 3,4; et/ou dans laquelle X représente un nombre d'au moins 2,2, de préférence d'au moins 2,4, de préférence d'au moins 2,6, de préférence d'au moins 2,8, de préférence d'au moins 3.

6. Composition adhésive de polyuréthane selon une quelconque revendication précédente, dans laquelle le composant polyisocyanate (i) comprend un ou plusieurs composés polyisocyanate choisis dans le groupe des polyisocyanates contenant de 2 à 10 fonctions isocyanate par molécule.

7. Composition adhésive de polyuréthane selon la revendication 6, dans laquelle le composant polyisocyanate (i) comprend un mélange d'un diisocyanate et d'au moins un polyisocyanate présentant une moyenne d'au moins 3 fonctions isocyanate par molécule.

8. Composition adhésive de polyuréthane selon la revendication 6 ou la revendication 7, dans laquelle le composant polyisocyanate (i) comprend un diisocyanate trimérisé de la formule $D(R-NCO)_3$, dans laquelle D représente une structure cyclique choisie parmi l'isocyanurate et l'iminooxadiazindione et des mélanges de ceux-ci, et chaque R représente indépendamment un groupe alkylène à chaîne droite, ramifié ou cyclique présentant de 2 à 15 atomes de carbone ou un groupe aryle présentant de 6 à 20 atomes de carbone, de préférence dans laquelle le au moins un polyisocyanate est du diisocyanate trimérisé d'hexaméthylène.

9. Composition adhésive de polyuréthane selon une quelconque revendication précédente, dans laquelle le au moins un composé (ii) comprend une fraction oléfine en tant que groupe fonctionnel qui est durcissable par polymérisation radicalaire ; de préférence dans laquelle le au moins un composé (ii) est choisi parmi les esters d'acrylate substitués par hydroxy, les esters de méthacrylate substitués par hydroxy et des mélanges de ceux-ci ; de préférence dans laquelle le au moins composé (ii) est choisi parmi les esters de méthacrylate substitués par hydroxy-alkyle en $(C_2-C_{20})$, le monométhacrylate polyalcoxylé contenant 2-10 fonctions éther et des mélanges de ceux-ci ; plus préférentiellement dans laquelle le composé (ii) est choisi parmi le méthacrylate de 2-hydroxypropyle, le méthacrylate d'hydroxyéthyle et des mélanges de ceux-ci.

10. Composition adhésive de polyuréthane selon une quelconque revendication précédente, dans laquelle le au moins un composé (iii) comprend ou consiste en un ou plusieurs alcools aliphatiques en $C_1-C_{30}$, de préférence des alcools aliphatiques en $C_1-C_{18}$ linéaires, ramifiés ou cycliques, de préférence des alcools aliphatiques en $C_1-C_{12}$ linéaires, ramifiés ou cycliques, de préférence des alcools aliphatiques en $C_3-C_{12}$ ramifiés, ou des alcools aliphatiques en $C_6-C_{18}$ ramifiés.

11. Composition adhésive de polyuréthane selon une quelconque revendication précédente, dans laquelle le au moins un composé (iii) comprend ou consiste en un ou plusieurs photo-initiateurs substitués par hydroxy.

12. Composition adhésive de polyuréthane selon une quelconque revendication précédente, dans laquelle le composant polymère (A) est choisi parmi les polyéthers à terminaison hydroxy, les polyesters à terminaison hydroxy, les polyéthers à terminaison amine et les polyesters à terminaison amine, de préférence dans laquelle le composant polymère est un polyéther à terminaison hydroxy ; de préférence dans laquelle le polyéther à terminaison hydroxy est choisi parmi les dérivés alcoxylés de composés comprenant de deux à cinq groupes hydroxy, ou des mélanges de ceux-ci ; de préférence dans laquelle le polyéther à terminaison hydroxy comprend des dérivés alcoxylés d'éthylène glycol, de propylène glycol, de butane-1,4-diol, de glycérol, de triméthylolpropane, d'érythritol, de pentaérythritol, de pentane-1,2,4,5-tétrol, de dextrose et de sorbitol, de préférence dans laquelle les dérivés alcoxylés sont des dérivés éthoxylés, des dérivés propoxylés ou des dérivés éthoxylés-co-propoxylés.

13. Composition adhésive de polyuréthane selon une quelconque revendication précédente, dans laquelle le composant polymère présente un poids moléculaire moyen en poids dans la plage de 1 000 à 50 000 Daltons, de préférence dans la plage de 1 000 à 20 000 Daltons, de préférence dans la plage de 1500 à 10 000 Daltons ; et/ou dans laquelle le composant polymère présente un poids équivalent par groupe fonctionnel nucléophile de 200 à 5 000, de préférence de 500 à 2 000, de préférence de 1 000 à 2 000.

14. Procédé de préparation d'une composition adhésive de polyuréthane comprenant :

(a) une première étape de réaction de :

(i) un composant polyisocyanate présentant une moyenne d'au moins Y fonctions isocyanate par molécule, dans lequel Y représente un nombre dans la plage de 1,8 à 6 ; avec
(ii) au moins un composé comprenant un groupe fonctionnel durcissable par polymérisation radicalaire et comprenant en outre un groupe fonctionnel nucléophile contenant un atome d'hydrogène actif ; et
(iii) au moins un composé comprenant un groupe fonctionnel nucléophile contenant un atome d'hydrogène actif et qui n'inclut pas de groupe fonctionnel durcissable par polymérisation radicalaire ;

dans lequel le degré total de substitution du polyisocyanate par les composés (ii) et (iii) est d'au moins 0,2 et inférieur ou égal au moins élevé parmi 0,3 Y et 0,8, pour former un composant de réticulation (B) ;
(b) une seconde étape de combinaison du produit de l'étape (i) avec un composant polymère (A) présentant un poids moléculaire moyen en poids dans la plage de 500 à 100 000 Daltons, et contenant une moyenne par molécule de X groupes fonctionnels nucléophiles contenant un atome d'hydrogène actif, dans lequel X représente un nombre supérieur à 2.

15. Produit médical adhésif comprenant une composition adhésive de polyuréthane commutable telle que définie dans l'une quelconque des revendications 1 à 13 disposée entre un premier film support et une pellicule antiadhésive ; de préférence dans lequel le premier film support est translucide aux UV, facultativement dans lequel une couche amovible opaque aux UV est stratifiée sur le premier film support sur la surface opposée à la composition adhésive.

Figure 1

Figure 2

Figure 3

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5032637 A
- US 5352516 A
- US 4331576 A
- US 5182323 A
- US 20130123678 A
- WO 2010129299 A
- WO 2013066401 A
- EP 0863775 A
- US 6184264 B
- US 6610762 B
- US 4999242 A
- US 5955512 A
- WO 2016124339 A1